(19)

Europäisches Patentamt

European Patent Office

Office européen des brevets

(11) **EP 4 484 444 A1**

(12) **EUROPEAN PATENT APPLICATION**

(43) Date of publication:
**01.01.2025   Bulletin 2025/01**

(21) Application number: **23306041.7**

(22) Date of filing: **28.06.2023**

(51) International Patent Classification (IPC):
**C07K 14/81** (2006.01)   **A61K 38/57** (2006.01)
**A61P 19/02** (2006.01)   **A61P 35/00** (2006.01)

(52) Cooperative Patent Classification (CPC):
**A61P 35/00; A61K 38/57; A61P 19/02;**
**C07K 14/81; C07K 14/8146**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **Centre National de la Recherche Scientifique**
  **75016 Paris (FR)**
• **UNIVERSITE CLAUDE BERNARD - LYON 1**
  **69100 Villeurbanne (FR)**

(72) Inventors:
• **VADON - LE GOFF, Sandrine**
  **69230 Saint Genis Laval (FR)**
• **MOALI, Catherine**
  **69230 Saint-Genis Laval (FR)**

(74) Representative: **Novagraaf Technologies**
**2 rue Sarah Bernhardt**
**CS90017**
**92665 Asnières-sur-Seine Cedex (FR)**

Remarks:
The complete document including Reference
Table(s) and the Sequence Listing(s) can be
downloaded from the EPO website

(54) **PROTEIN AND FRAGMENTS THEREOF, AND THEIR USE**

(57)    The present invention relates to peptide comprising the amino acid sequence SEQ ID NO :1, provided that said peptide does not consist in SEQ ID NO: 2, or SEQ ID NO: 3 or any variant thereof having at least 36% identity with SEQ ID NO: 1 or SEQ ID NO: 3, provided that the underlined cysteines (C) in SEQ ID NO: 1 are conserved, and that the variant does not contain another cysteine, and that said variant retains the ability to bind and/or inhibit a BMP-1/tolloid-like protease or BTP.

The use of the peptide in therapy is also disclosed.

**EP 4 484 444 A1**

**Description**

The invention relates to a protein and fragments thereof, for their use for treating pathologies.

BMP-1 (bone morphogenetic protein-1) and mTLL-1 (mammalian tolloid-like 1) are two essential extracellular metalloproteases for mammalian life. Mice lacking the Bmp1 or Tll1 gene die at mid-gestation or around birth with several defects affecting their skeleton, gut or heart. Patients with mutations in their BMP1 gene suffer from Osteogenesis Imperfecta. These severe phenotypes are linked to the crucial contribution of the two enzymes to extracellular matrix assembly and growth factor activation. Importantly, they proteolytically remove the C-terminal propeptide of fibrillar procollagens and, in some cases, their N-terminal propeptide to decrease the solubility of collagen monomers and trigger fibrillogenesis. They also activate lysyl oxidases LOX and LOXL to promote collagen and elastin cross-linking, process laminin 332 and collagen VII to allow basement membrane assembly and cleave dentin matrix protein-1 and dentin sialophosphoprotein to favor calcium fixation during bone and tooth mineralization. Other critical functions include the activation of BMP-2/4/11 growth factors through the cleavage of their antagonists or propeptides and the enhancement of TGF-β signalling through various cooperative mechanisms. Together with mTLL-2, which has been more specifically involved in the control of muscle growth through the cleavage of myostatin but seems to have a more limited tissue distribution, BMP-1 and mTLL-1 form the BTP (BMP-1/tolloid-like proteinases) family that is included in the astacin-like subgroup of metzincins. BMP-1 is often expressed together with a splice variant called mTLD (mammalian tolloid) that can be considered as a distinct and additional BTP enzyme with slightly different properties.

Strict control of BTP activities in time and space is also required for tissue homeostasis and regeneration in adults. For example, BTP activity is needed for bone remodelling and BMP-1 dysregulation has been implicated in osteoporosis and osteoarthritis. In soft tissues, skin wound healing is severely impaired in mice in the absence of Bmp1 and Tll1. Interestingly also, BTP expression is generally increased in fibrotic and scarring processes while the inhibition of BTP activity tends to normalize tissue repair. In addition, polymorphisms in TLL1 were associated with the complications of some metabolic disorders (type 2 diabetes and nonalcoholic fatty liver disease) and BMP-1 activity was suggested to play a role in lipid metabolism and maternal diabetes. Finally, high BMP1 expression is a factor of poor prognosis in several cancer types but the secretion of active BMP-1 by tumor cells in PDAC (pancreatic ductal adenocarcinoma) could be protective against tumor growth and metastasis.

Despite these important pathophysiological functions, the regulatory mechanisms controlling BTP activity have remained elusive. Some natural inhibitors have been described but these were either not specific to BTPs (α2-macroglobulin), not conserved in mammals (sizzled) or highly controversial with divergent effects observed in different laboratories (sFRP-2 and BMP-4). Interestingly, this lack of specific inhibitors in mammals is associated with the existence of several enhancing proteins which, in most cases, seem to work in a substrate-specific manner to specifically promote the cleavage of one type of substrates (i.e. fibrillar collagens, LOX, myostatin and chordin).

Fibrosis is a disorder characterised by excessive synthesis and deposition of extracellular matrix (ECM), leading to pathological scarring and loss of function, which can result in the patient's death when vital organs are affected (heart, liver, lung, etc.) or in significant functional and cosmetic handicaps if external organs (skin, cornea) are affected. Similarly, some tumours may be surrounded by a gangue of extracellular matrix (desmoplasia) that prevents the penetration of immune cells and anti-cancer drugs. A typical case is pancreatic cancer, which is associated with a very low survival rate.

In both these cases, fibrillar collagens are the main components of the pathological ECM. The imbalance between their synthesis and degradation is one of the main factors favouring the development of fibrosis, and inhibiting their biosynthesis is a promising therapeutic approach for treating or preventing the onset of fibrosis and stromal reactions around tumours. Synthesised in the form of soluble precursors, collagens must undergo proteolytic maturation before they can be assembled into fibres. This limiting step is regulated by BTPs (BMP-1, mTLD, mTLL-1 and to a lesser extent mTLL-2) and the PCPE-1 protein (procollagen C-proteinase enhancer 1), which is a highly efficient and specific activator of the maturation reaction. Several of these proteins are over-expressed in numerous fibrotic processes and in the stroma surrounding pancreatic tumours. The gain of function associated with a decrease in their expression or activity has also been demonstrated in several animal models of fibrosis.

BTPs potentially have other pro-tumour activities via activation of TGF-β signalling and regulation of angiogenesis. They have been associated with poor survival in many cancers. In addition, they activate a muscle growth inhibitor, myostatin, and the benefit of reducing their activity to promote muscle growth is well established. Finally, BTPs reduce the uptake of LDL-cholesterol by cells via cleavage of the LDLR receptor, which could give them a role in metabolic diseases (unproven to date because mouse models are unsuitable). Small molecule inhibitors of BTPs already exist, mainly developed by manufacturers such as Pfizer and Glaxo (US7214694; WO2017/006296). However, their specificity towards meprins and other metalloproteases is not always optimal. The Sizzled protein, found in xenopus and zebrafish but not in mammals, has also been described as a highly potent and selective inhibitor of BTPs, but may pose immunogenicity problems if injected into humans.

Thus, there is a need to provide a new efficient BTP inhibitor.

More recently, antibodies capable of blocking the various BTP enzymes have been developed (WO2008/011193A2; WO2013/163479A1; WO2022/024034A1) suggesting that it is relevant to target these proteases in the context of fibrosis and muscle disorders.

The invention intends to provide a new type of efficient and specific inhibitors against BTPs.

One aim of the invention is to provide new peptides modulating BTP.

Another aim of the invention is to provide a new drug for treating pathologies involving deregulation of BTP.

The invention relates to

- a peptide comprising, consisting essentially, or consisting of the amino acid sequence SEQ ID NO:1

> CGGILTGESGFIGSEGFPGVYPPNSKCTWKITVPEGKVVVLNFRFIDLESDNLCRYDFVDVY
> NGHANGQRIGRFCGTFRPGALVSSGNKMMVQMISDANTAGNGFMAMFSAA (SEQ ID
> NO:1),

provided that said peptide does not consist in SEQ ID NO: 2, or SEQ ID NO: 3,

- or any variant thereof having at least 36% identity with SEQ ID NO: 1 or SEQ ID NO: 3, provided

> i) that the underlined cysteines (C) in SEQ ID NO: 1 are conserved, and that the variant does not contain another cysteine, and
> ii) that said variant retains the ability to bind and/or inhibit a BMP-1/tolloid-like protease or BTP.

The invention is based on the unexpected observation made by the inventors that the CUB1 domain of the PCPE-2 protein, of SEQ ID NO: 1, is able to interact with and inhibit proteolytic activity of BTP.

The above peptides, or their variants thereof, are novel. However, peptides corresponding to the native procollagen C-proteinase enhancer 2 (PCPE-2) protein (SEQ ID NO: 2),

> QSPERPVFTCGGILTGESGFIGSEGFPGVYPPNSKCTWKITVPEGKVVVLNFRFIDLESD
> NLCRYDFVDVYNGHANGQRIGRFCGTFRPGALVSSGNKMMVQMISDANTAGNGFMAMFS
> AAEPNERGDQYCGGLLDRPSGSFKTPNWPDRDYPAGVTCVWHIVAPKNQLIELKFEKFDV
> ERDNYCRYDYVAVFNGGEVNDARRIGKYCGDSPPAPIVSERNELLIQFLSDLSLTADGFIGH
> YIFRPKKLPTTTEQPVTTTFPVTTGLKPTVALCQQKCRRTGTLEGNYCSSDFVLAGTVITTITR
> DGSLHATVSIINIYKEGNLAIQQAGKNMSARLTVVCKQCPLLRRGLNYIIMGQVGEDGRGKIM
> PNSFIMMFKTKNQKLLDALKNKQC,

or the protein containing the signal peptide (SEQ ID NO: 3)

> MRGANAWAPLCLLLAAATQLSRQQSPERPVFTCGGILTGESGFIGSEGFPGVYPPNSKC
> TWKITVPEGKVVVLNFRFIDLESDNLCRYDFVDVYNGHANGQRIGRFCGTFRPGALVSSGN
> KMMVQMISDANTAGNGFMAMFSAAEPNERGDQYCGGLLDRPSGSFKTPNWPDRDYPAG
> VTCVWHIVAPKNQLIELKFEKFDVERDNYCRYDYVAVFNGGEVNDARRIGKYCGDSPPAPIV
> SERNELLIQFLSDLSLTADGFIGHYIFRPKKLPTTTEQPVTTTFPVTTGLKPTVALCQQKCRRT
> GTLEGNYCSSDFVLAGTVITTITRDGSLHATVSIINIYKEGNLAIQQAGKNMSARLTVVCKQCP
> LLRRGLNYIIMGQVGEDGRGKIMPNSFIMMFKTKNQKLLDALKNKQC

are excluded from the scope of the invention.

SEQ ID NO: 1 corresponds to the amino acid sequence of the Complement, Uegf, BMP-1 domain 1 (CUB1) of PCPE-2. PCPE-2 is currently described as an enhancer of BTP, as well as the protein PCPE-1 with which it shares 43 % sequence identity. Both proteins comprise 2 CUB domains (CUB1 and CUB2) followed by a linker and a netrin-like (NTR) domain.

PCPE-1 has been described to efficiently stimulates the C-terminal maturation of fibrillar collagens by BTPs. In mice lacking PCPE-1 (Pcolce-null mice), aberrantly-shaped collagen fibrils were observed in bones and tendons and tissue mechanical properties were found to be altered. Biochemical and structural studies have revealed that PCPE-1 exerts its procollagen C-proteinase (PCP) enhancing activity by directly binding the procollagen substrate in the C-terminal globular region (C-propeptide). This tight PCPE-1/procollagen interaction induces a local conformational change in the vicinity of the cleavage site, that facilitates the cleavage of the compact procollagen trimer by BTPs.

PCPE-2 has been much less studied but, as it was also found to enhance the in vitro cleavage of procollagen II by BMP-1 and mTLL-1, the current view is that PCPE-2 behaves like PCPE-1 in terms of enhancing procollagen maturation.

The inventors have identified that CUB1 domain of PCPE-2 bind and/or inhibit a BTP, in particular BMP-1.

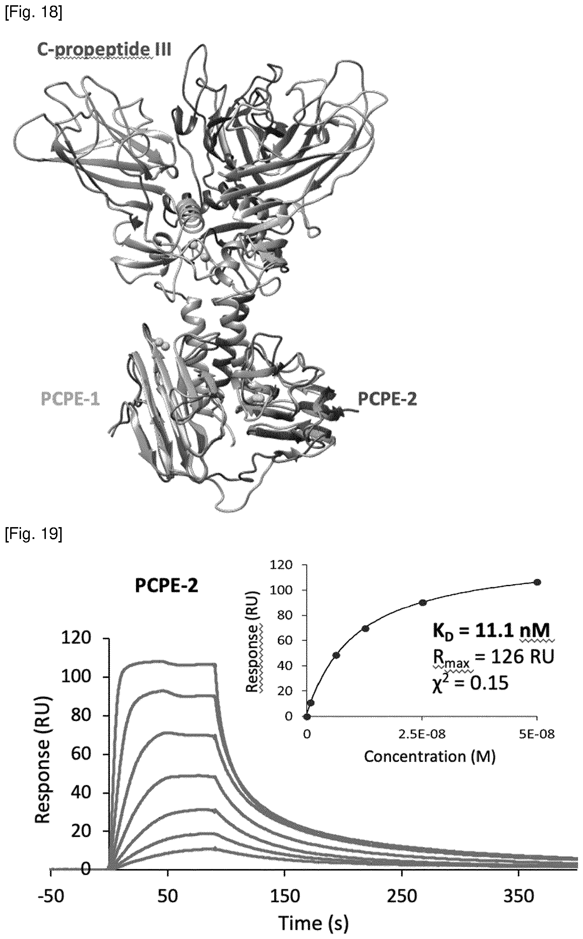

Variants of the CUB1 domain are also covered by the present invention, in particular variants sharing at least 36% identity with SEQ ID NO: 1. These variants can therefore share 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with SEQ ID NO: 1. It is however advantageous that these variants retain the cysteines in position 1, 27, 54 and 75 of SEQ ID NO: 1, or similar positions, such that these cysteine retain the ability to form two disulfide bridges, in order to harbor a three dimensional structure as depicted in Figure 18. Moreover, SEQ ID NO: 1 and all the variants mentioned above retain the ability to interact with/bind or inhibit, or both a BTP. Therefore, if a peptide or a variant retaining the 4 above mentioned cysteines does not interact with a BTP, or does not inhibit a BTP, this peptide or variant thereof is not covered by the invention.

The substitutions will be determined for each amino acid. For instance, if the amino acid to be substituted is an acidic amino acid, the amino acid will be substituted by an Alanine, or a basic amino acid. Of course, the skilled person could easily choose the appropriated amino acid to be used for the substation.

Interaction between the peptides according to the invention and a BTP can be evaluated by any technique well known in the art, such as surface plasmon resonance, ELISA, co-immunoprecipitation assay, or pull-down assays. The skilled person could easily choose the most suitable technique.

Inhibition of BTP activity can be evaluated as disclosed in the Examples.

The above-mentioned peptides can be produced by using a nucleic acid coding said peptides, in an in vitro or an in vivo expression system for expressing proteins, followed by a purification step. These techniques are well known in the art, and the skilled person could easily choose the most suitable technique.

In order to avoid any insolubility or stability issues of the peptides, the inventors produced the peptides as defined above as disclosed in the examples.

Advantageously, the invention relates to the peptide as defined above, wherein at least one of the amino acids in position 15, 16, 19, 20, 23, 24, 44, 45, 47, 48, 50, 51, 52, 53, 55, 56, 76, 77, 78, 79, 98, 99, 100, 101, 102 and 103 of SEQ ID NO: 1 is substituted by any other amino acid, preferably wherein at least one of the amino acids in position 44, 78 and 79 of SEQ ID NO: 1 is substituted by any other amino acid.

The inventors have shown that some mutations, in particular substitutions, within SEQ ID NO: 1 inhibit the ability of PCPE-2 derived peptides to enhance the cleavage of procollagen III, but does not affect the properties of inhibition of BTPs.

By "substituted by any other amino acid", it is meant in the invention a substitution by any of the amino acids which is not the amino acid of SEQ ID NO:1 at the determined position, and, except cysteine.

For instance, position 15 of SEQ ID NO: 1, which is a Glutamic acid, can be substituted by any amino acid, except cysteine and glutamic acid.

Advantageously, the invention relates to the peptide as defined above, wherein said peptide comprises, consists essentially of, or consists of both amino acid sequences SEQ ID NO: 1 and SEQ ID NO: 4.

More advantageously, peptides comprising both CUB1 domain of PCPE-2 (SEQ ID NO: 1) and CUB2 domain of PCPE-2, i.e. the amino acid sequence SEQ ID NO: 4, CGGLLDRPSGSFKTPNWPDRDYPAGVTCVWHIVAPKNQLIE LKFEKFDVERDNYCRYDYV AVFNGGEVNDARRIGKYCGDSPPAPIVSERNELLIQFLSDLSLTADGFIGHYIFR, are particularly active for inhibiting a BTP.

Advantageously, the invention relates to the peptide as defined above, wherein at least one of the amino acids in position 21, 52, 53, 54, 56, 57, 70, 73, 74, 75, 76, 77, 80, 81, 82, 84, 86, 101, 102, 103, 104 and 106 of SEQ ID NO: 4 is substituted by any other amino acid, preferably the amino acid in position 101 of SEQ ID NO: 4 is substituted by any amino acid.

The definitions mentioned above for CUB1 domain mutations apply mutatis mutandis, i.e. each of the above mentioned amino acids can be substituted by any amino acid, except by the original amino acid and a cysteine.

More advantageously, the invention relates to the peptide as defined above, wherein SEQ ID NO: 1 and SEQ ID NO: 4 are directly linked by a linker.

As for the natural PCPE-2 protein, it is advantageous that the CUB domains (CUB1 and CUB2, in their WT or mutant forms) be separated by a linker.

In one embodiment, it is only important that SEQ ID NO: 1 and SEQ ID NO: 4, in the wild type form or a mutant form be present in order to provide to the peptide the ability to inhibit BTP.

More advantageously, the invention relates to the peptide as defined above, wherein, from the N-terminus to the C-terminus of the peptide, the sequence of the peptide comprises SEQ ID NO: 1, the C-terminus part of SEQ ID NO: 1 is directly linked to the N-terminus part of the linker, and the C-terminus part of the linker is directly linked to the N-terminus part of SEQ ID NO: 4.

An advantageous configuration of the peptides defined above is that from the N-terminus to the C-terminus of the peptide that CUB1 domain be followed by a linker, the linker itself being followed by CUB2 domain. This corresponds to the configuration found in the PCPE-2 protein.

More advantageously, the invention relates to the peptide as defined above, wherein the linker is chosen from the group comprising SEQ ID NO: 5 or SEQ ID NO: 6 or (GS)n wherein n varies from 1 to 20 or (GGGS)m wherein m varies from 1 to 10.

The above linker sequences confer to the peptide according to the invention a flexibility such that the peptide exerts an inhibitory effect on a BTP.

More advantageously, the invention relates to the peptide as defined above, wherein the peptide comprises a sequence able to interact with one of the BTPs.

The above defined sequence able to interact with at least one of the BTPs is particularly advantageous, since it increases the interaction between the peptide according to the invention and therefore facilitates the inhibition of the BTP with which the peptide interacts with.

More advantageously, the invention relates to the peptide as defined above, the peptide comprising, consisting essentially or consisting of one of the following sequences: SEQ ID NO: 7 to SEQ ID NO: 59.

These peptides are particularly advantageous and are able to inhibit a BTP. These peptides are the following ones:

[Table 1]

| PCP E-2-3C | ETGHHHHHHLEVLFQGPASQSPERPVFTCGGILTGESGFIGSEGFPGVYP PNSKCTWKITVPEGKVVVLNFRFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFRPKLEVLFQGPKLPTTTEQPVTTTFPVTTGLKPTVALCQ QKCRRTGTLEGNYCSSDFVLAGTVITTITRDGSLHATVSIINIYKEGNLAIQQ AGKNMSARLTVVCKQCPLLRRGLNYIIMGQVGEDGRGKIMPNSFIMMFKTK NQKLLDALKNKQC | SEQ ID NO: 7 |
|---|---|---|
| C1-C2* | GPASQSPERPVFTCGGILTGESGFIGSEGFPGVYPPNSKCTWKITVPEGKV VVLNFRFIDLESDNLCRYDFVDVYNGHANGQRIGRFCGTFRPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDLSLTADGFIGHYIFRPKLE VLFQ | SEQ ID NO: 8 |
| C1-C2-mVe nus | ETGHHHHHHQSPERPVFTCGGILTGESGFIGSEGFPGVYPPNSKCTWKIT VPEGKVVVLNFRFIDLESDNLCRYDFVDVYNGHANGQRIGRFCGTFRPGA LVSSGNKMMVQMISDANTAGNGFMAMFSAAEPNERGDQYCGGLLDRPS GSFKTPNWPDRDYPAGVTCVWHIVAPKNQLIELKFEKFDVERDNYCRYDY VAVFNGGEVNDARRIGKYCGDSPPAPIVSERNELLIQFLSDLSLTADGFIGH YIFRPKKLPTTTEQPVTTTFPVTTGLKPTVALASMVSKGEELFTGVVPILVEL DGDVNGHKFSVSGEGEGDATYGKLTLKLICTTGKLPVPWPTLVTTLGYGL QCFARYPDHMKQHDFFKSAMPEGYVQERTIFFKDDGNYKTRAEVKFEGD TLVNRIELKGIDFKEDGNILGHKLEYNYNSHNVYITADKQKNGIKANFKIRHNI EDGGVQLADHYQQNTPIGDGPVLLPDNHYLSYQSKLSKDPNEKRDHMVLL EFVTAAGITLGMDELY | SEQ ID NO: 9 |

| | | |
|---|---|---|
| MBP-C1-C2 | ETGTKIEEGKLVIWINGDKGYNGLAEVGKKFEKDTGIKVTVEHPDKLEEKFP QVAATGDGPDIIFWAHDRFGGYAQSGLLAEITPAAAFQDKLYPFTWDAVRY NGKLIAYPIAVEALSLIYNKDLLPNPPKTWEEIPALDKELKAKGKSALMFNLQ EPYFTWPLIAADGGYAFKYAAGKYDIKDVGVDNAGAKAGLTFLVDLIKNKH MNADTDYSIAEHAFNHGETAMTINGPWAWSNIDTSAVNYGVTVLPTFKGQ PSKPFVGVLSAGINAASPNKELAKEFLENYLLTDEGLEAVNKDKPLGAVALK SYEEELVKDPRVAATMENAQKGEIMPNIPQMSAFWYAVRTAVINAASGRQ TVDAALAAAQTNAAALEVLFQGPASQSPERPVFTCGGILTGESGFIGSEGF PGVYPPNSKCTWKITVPEGKVVVLNFRFIDLESDNLCRYDFVDVYNGHAN GQRIGRFCGTFRPGALVSSGNKMMVQMISDANTAGNGFMAMFSAAEPNE RGDQYCGGLLDRPSGSFKTPNWPDRDYPAGVTCVWHIVAPKNQLIELKFE KFDVERDNYCRYDYVAVFNGGEVNDARRIGKYCGDSPPAPIVSERNELLIQ FLSDLSLTADGFIGHYIFRPKKLEHHHHHH | SEQ ID NO: 10 |
| C1-C2 | GPASQSPERPVFTCGGILTGESGFIGSEGFPGVYPPNSKCTWKITVPEGKV VVLNFRFIDLESDNLCRYDFVDVYNGHANGQRIGRFCGTFRPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDLSLTADGFIGHYIFRPKKL EHHHHHH | SEQ ID NO: 11 |
| MBP-C1(F 78A)-C2 | ETGTKIEEGKLVIWINGDKGYNGLAEVGKKFEKDTGIKVTVEHPDKLEEKFP QVAATGDGPDIIFWAHDRFGGYAQSGLLAEITPAAAFQDKLYPFTWDAVRY NGKLIAYPIAVEALSLIYNKDLLPNPPKTWEEIPALDKELKAKGKSALMFNLQ EPYFTWPLIAADGGYAFKYAAGKYDIKDVGVDNAGAKAGLTFLVDLIKNKH MNADTDYSIAEHAFNHGETAMTINGPWAWSNIDTSAVNYGVTVLPTFKGQ PSKPFVGVLSAGINAASPNKELAKEFLENYLLTDEGLEAVNKDKPLGAVALK SYEEELVKDPRVAATMENAQKGEIMPNIPQMSAFWYAVRTAVINAASGRQ TVDAALAAAQTNAAALEVLFQGPASQSPERPVFTCGGILTGESGFIGSEGF PGVYPPNSKCTWKITVPEGKVVVLNFRFIDLESDNLCRYDFVDVYNGHAN<br><br>GQRIGRFCGTARPGALVSSGNKMMVQMISDANTAGNGFMAMFSAAEPNE RGDQYCGGLLDRPSGSFKTPNWPDRDYPAGVTCVWHIVAPKNQLIELKFE KFDVERDNYCRYDYVAVFNGGEVNDARRIGKYCGDSPPAPIVSERNELLIQ FLSDLSLTADGFIGHYIFRPKKLEHHHHHH | SEQ ID NO: 12 |

| | | |
|---|---|---|
| C1(F 78A)-C2 | GPASQSPERPVFTCGGILTGESGFIGSEGFPGVYPPNSKCTWKITVPEGKV VVLNFRFIDLESDNLCRYDFVDVYNGHANGQRIGRFCGTARPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDLSLTADGFIGHYIFRPKKL EHHHHHH | SEQ ID NO: 13 |
| MBP-C1(R 79D)-C2 | ETGTKIEEGKLVIWINGDKGYNGLAEVGKKFEKDTGIKVTVEHPDKLEEKFP QVAATGDGPDIIFWAHDRFGGYAQSGLLAEITPAAAFQDKLYPFTWDAVRY NGKLIAYPIAVEALSLIYNKDLLPNPPKTWEEIPALDKELKAKGKSALMFNLQ EPYFTWPLIAADGGYAFKYAAGKYDIKDVGVDNAGAKAGLTFLVDLIKNKH MNADTDYSIAEHAFNHGETAMTINGPWAWSNIDTSAVNYGVTVLPTFKGQ PSKPFVGVLSAGINAASPNKELAKEFLENYLLTDEGLEAVNKDKPLGAVALK SYEEELVKDPRVAATMENAQKGEIMPNIPQMSAFWYAVRTAVINAASGRQ TVDAALAAAQTNAAALEVLFQGPASQSPERPVFTCGGILTGESGFIGSEGF PGVYPPNSKCTWKITVPEGKVVVLNFRFIDLESDNLCRYDFVDVYNGHAN GQRIGRFCGTFDPGALVSSGNKMMVQMISDANTAGNGFMAMFSAAEPNE RGDQYCGGLLDRPSGSFKTPNWPDRDYPAGVTCVWHIVAPKNQLIELKFE KFDVERDNYCRYDYVAVFNGGEVNDARRIGKYCGDSPPAPIVSERNELLIQ FLSDLSLTADGFIGHYIFRPKKLEHHHHHH | SEQ ID NO: 14 |
| C1(R 79D)-C2 | GPASQSPERPVFTCGGILTGESGFIGSEGFPGVYPPNSKCTWKITVPEGKV VVLNFRFIDLESDNLCRYDFVDVYNGHANGQRIGRFCGTFDPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDLSLTADGFIGHYIFRPKKL EHHHHHH | SEQ ID NO: 15 |

| | | |
|---|---|---|
| MBP-C1-C2(L 101A ) | ETGTKIEEGKLVIWINGDKGYNGLAEVGKKFEKDTGIKVTVEHPDKLEEKFP QVAATGDGPDIIFWAHDRFGGYAQSGLLAEITPAAAFQDKLYPFTWDAVRY NGKLIAYPIAVEALSLIYNKDLLPNPPKTWEEIPALDKELKAKGKSALMFNLQ EPYFTWPLIAADGGYAFKYAAGKYDIKDVGVDNAGAKAGLTFLVDLIKNKH MNADTDYSIAEHAFNHGETAMTINGPWAWSNIDTSAVNYGVTVLPTFKGQ PSKPFVGVLSAGINAASPNKELAKEFLENYLLTDEGLEAVNKDKPLGAVALK SYEEELVKDPRVAATMENAQKGEIMPNIPQMSAFWYAVRTAVINAASGRQ TVDAALAAAQTNAAALEVLFQGPASQSPERPVFTCGGILTGESGFIGSEGF PGVYPPNSKCTWKITVPEGKVVVLNFRFIDLESDNLCRYDFVDVYNGHAN GQRIGRFCGTFRPGALVSSGNKMMVQMISDANTAGNGFMAMFSAAEPNE RGDQYCGGLLDRPSGSFKTPNWPDRDYPAGVTCVWHIVAPKNQLIELKFE KFDVERDNYCRYDYVAVFNGGEVNDARRIGKYCGDSPPAPIVSERNELLIQ FLSDASLTADGFIGHYIFRPKKLEHHHHHH | SEQ ID NO: 16 |
| C1-C2(L 101A ) | GPASQSPERPVFTCGGILTGESGFIGSEGFPGVYPPNSKCTWKITVPEGKV VVLNFRFIDLESDNLCRYDFVDVYNGHANGQRIGRFCGTFRPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDASLTADGFIGHYIFRPKKL EHHHHHH | SEQ ID NO: 17 |
| MBP-C1(R 44A)-C2(L 101A ) | ETGTKIEEGKLVIWINGDKGYNGLAEVGKKFEKDTGIKVTVEHPDKLEEKFP QVAATGDGPDIIFWAHDRFGGYAQSGLLAEITPAAAFQDKLYPFTWDAVRY NGKLIAYPIAVEALSLIYNKDLLPNPPKTWEEIPALDKELKAKGKSALMFNLQ EPYFTWPLIAADGGYAFKYAAGKYDIKDVGVDNAGAKAGLTFLVDLIKNKH MNADTDYSIAEHAFNHGETAMTINGPWAWSNIDTSAVNYGVTVLPTFKGQ PSKPFVGVLSAGINAASPNKELAKEFLENYLLTDEGLEAVNKDKPLGAVALK<br><br>SYEEELVKDPRVAATMENAQKGEIMPNIPQMSAFWYAVRTAVINAASGRQ TVDAALAAAQTNAAALEVLFQGPASQSPERPVFTCGGILTGESGFIGSEGF PGVYPPNSKCTWKITVPEGKVVVLNFAFIDLESDNLCRYDFVDVYNGHANG QRIGRFCGTFRPGALVSSGNKMMVQMISDANTAGNGFMAMFSAAEPNER GDQYCGGLLDRPSGSFKTPNWPDRDYPAGVTCVWHIVAPKNQLIELKFEK FDVERDNYCRYDYVAVFNGGEVNDARRIGKYCGDSPPAPIVSERNELLIQF LSDASLTADGFIGHYIFRPKKLEHHHHHH | SEQ ID NO: 18 |

| | | |
|---|---|---|
| C1(R 44A)-C2(L 101A ) | GPASQSPERPVFTCGGILTGESGFIGSEGFPGVYPPNSKCTWKITVPEGKV VVLNFAFIDLESDNLCRYDFVDVYNGHANGQRIGRFCGTFRPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDASLTADGFIGHYIFRPKKL EHHHHHH | SEQ ID NO: 19 |
| MBP-C1(R 44A, F78A ,R79 D)-C2(L 101A ) | ETGTKIEEGKLVIWINGDKGYNGLAEVGKKFEKDTGIKVTVEHPDKLEEKFP QVAATGDGPDIIFWAHDRFGGYAQSGLLAEITPAAAFQDKLYPFTWDAVRY NGKLIAYPIAVEALSLIYNKDLLPNPPKTWEEIPALDKELKAKGKSALMFNLQ EPYFTWPLIAADGGYAFKYAAGKYDIKDVGVDNAGAKAGLTFLVDLIKNKH MNADTDYSIAEHAFNHGETAMTINGPWAWSNIDTSAVNYGVTVLPTFKGQ PSKPFVGVLSAGINAASPNKELAKEFLENYLLTDEGLEAVNKDKPLGAVALK SYEEELVKDPRVAATMENAQKGEIMPNIPQMSAFWYAVRTAVINAASGRQ TVDAALAAAQTNAAALEVLFQGPASQSPERPVFTCGGILTGESGFIGSEGF PGVYPPNSKCTWKITVPEGKVVVLNFAFIDLESDNLCRYDFVDVYNGHANG QRIGRFCGTADPGALVSSGNKMMVQMISDANTAGNGFMAMFSAAEPNER GDQYCGGLLDRPSGSFKTPNWPDRDYPAGVTCVWHIVAPKNQLIELKFEK FDVERDNYCRYDYVAVFNGGEVNDARRIGKYCGDSPPAPIVSERNELLIQF LSDASLTADGFIGHYIFRPKKLEHHHHHH | SEQ ID NO: 20 |
| C1(R 44A, F78A ,R79 D)-C2(L 101A ) | GPASQSPERPVFTCGGILTGESGFIGSEGFPGVYPPNSKCTWKITVPEGKV VVLNFAFIDLESDNLCRYDFVDVYNGHANGQRIGRFCGTADPGALVSSGN KMMVQMISDANTAGNGFMAMFSAAEPNERGDQYCGGLLDRPSGSFKTPN WPDRDYPAGVTCVWHIVAPKNQLIELKFEKFDVERDNYCRYDYVAVFNGG EVNDARRIGKYCGDSPPAPIVSERNELLIQFLSDASLTADGFIGHYIFRPKKL EHHHHHH | SEQ ID NO: 21 |
| C2-NTR | ETGHHHHHHCGGLLDRPSGSFKTPNWPDRDYPAGVTCVWHIVAPKNQLI ELKFEKFDVERDNYCRYDYVAVFNGGEVNDARRIGKYCGDSPPAPIVSER NELLIQFLSDLSLTADGFIGHYIFRPKKLPTTTEQPVTTTFPVTTGLKPTVALC QQKCRRTGTLEGNYCSSDFVLAGTVITTITRDGSLHATVSIINIYKEGNLAIQ QAGKNMSARLTVVCKQCPLLRRGLNYIIMGQVGEDGRGKIMPNSFIMMFK TKNQKLLDALKNKQC | SEQ ID NO: 22 |

(continued)

| | | |
|---|---|---|
| C2 | CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFR | SEQ ID NO: 23 |
| C1(F 78A) | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAA | SEQ ID NO: 24 |
| C1C2 -GS | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAGSGSGSGSGSCGGLLDRPSGSFKTPNWPDRDYPAGVT CVWHIVAPKNQLIELKFEKFDVERDNYCRYDYVAVFNGGEVNDARRIGKYC GDSPPAPIVSERNELLIQFLSDLSLTADGFIGHYIFR | SEQ ID NO: 25 |
| C1C2-GGG S | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAGGGSGGGSCGGLLDRPSGSFKTPNWPDRDYPAGVTCV WHIVAPKNQLIELKFEKFDVERDNYCRYDYVAVFNGGEVNDARRIGKYCG DSPPAPIVSERNELLIQFLSDLSLTADGFIGHYIFR | SEQ ID NO: 26 |
| C1(F 78A)-C2-GS | CGGILTGESGFIGSEGFPGVYPPNSKCTWKITVPEGKVVVLNFRFIDLESDN LCRYDFVDVYNGHANGQRIGRFCGTARPGALVSSGNKMMVQMISDANTA GNGFMAMFSAAGSGSGSGSGSCGGLLDRPSGSFKTPNWPDRDYPAGVT CVWHIVAPKNQLIELKFEKFDVERDNYCRYDYVAVFNGGEVNDARRIGKYC GDSPPAPIVSERNELLIQFLSDLSLTADGFIGHYIFR | SEQ ID NO: 27 |
| C1(F 78A)-C2-GGG S | CGGILTGESGFIGSEGFPGVYPPNSKCTWKITVPEGKVVVLNFRFIDLESDN LCRYDFVDVYNGHANGQRIGRFCGTARPGALVSSGNKMMVQMISDANTA GNGFMAMFSAAGGGSGGGSCGGLLDRPSGSFKTPNWPDRDYPAGVTCV WHIVAPKNQLIELKFEKFDVERDNYCRYDYVAVFNGGEVNDARRIGKYCG DSPPAPIVSERNELLIQFLSDLSLTADGFIGHYIFR | SEQ ID NO: 28 |

(continued)

| | | |
|---|---|---|
| PCP E-2_sh ort | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFRPKKLPTTTEQPVTTTF PVTTGLKPTVALCQQKCRRTGTLEGNYCSSDFVLAGTVITTITRDGSLHATV SIINIYKEGNLAIQQAGKNMSARLTVVCKQCPLLRRGLNYIIMGQVGEDGRG KIMPNSFIMMFKTKNQKLLDALKNKQC | SEQ ID NO: 29 |
| PCP E-2-3C_s hort | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFRPKLEVLFQGPKLPTTT EQPVTTTFPVTTGLKPTVALCQQKCRRTGTLEGNYCSSDFVLAGTVITTITR DGSLHATVSIINIYKEGNLAIQQAGKNMSARLTVVCKQCPLLRRGLNYIIMG QVGEDGRGKIMPNSFIMMFKTKNQKLLDALKNKQC | SEQ ID NO: 30 |
| C1-C2-mVe nus_ short | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFRPKKLPTTTEQPVTTTF PVTTGLKPTVALASMVSKGEELFTGVVPILVELDGDVNGHKFSVSGEGEGD ATYGKLTLKLICTTGKLPVPWPTLVTTLGYGLQCFARYPDHMKQHDFFKSA MPEGYVQERTIFFKDDGNYKTRAEVKFEGDTLVNRIELKGIDFKEDGNILGH KLEYNYNSHNVYITADKQKNGIKANFKIRHNIEDGGVQLADHYQQNTPIGD GPVLLPDNHYLSYQSKLSKDPNEKRDHMVLLEFVTAAGITLGMDELY | SEQ ID NO: 31 |

| | | |
|---|---|---|
| MBP-C1-C2_s hort | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFRPKKL | SEQ ID NO: 32 |
| MBP-C1-C2_s hort_ 2 | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFR | SEQ ID NO: 33 |
| C1-C2_s hort | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFRPKKL | SEQ ID NO: 34 |

| | | |
|---|---|---|
| C1-C2_s hort_2 | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFR | SEQ ID NO: 35 |
| MBP-C1(F 78A)-C2_s hort | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTARPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFRPKKL | SEQ ID NO: 36 |
| MBP-C1(F 78A)-C2_s hort_2 | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTARPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFR | SEQ ID NO: 37 |

EP 4 484 444 A1

| | | |
|---|---|---|
| C1(F 78A)-C2_s hort | CGGILTGESGFIGSEGFPGVYPPNSKCTWKITVPEGKVVVLNFRFIDLESDN LCRYDFVDVYNGHANGQRIGRFCGTARPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFRPKKL | SEQ ID NO: 38 |
| C1(F 78A)-C2_s hort2 | CGGILTGESGFIGSEGFPGVYPPNSKCTWKITVPEGKVVVLNFRFIDLESDN LCRYDFVDVYNGHANGQRIGRFCGTARPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFR | SEQ ID NO: 39 |
| MBP-C1(R 79D)-C2_s hort | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTFDPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFRPKKL | SEQ ID NO: 40 |

(continued)

| | | |
|---|---|---|
| MBP-C1(R 79D)-C2_s hort2 | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTFDPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFR | SEQ ID NO: 41 |
| C1(R 79D)-C2_s hort | CGGILTGESGFIGSEGFPGVYPPNSKCTWKITVPEGKVVVLNFRFIDLESDN LCRYDFVDVYNGHANGQRIGRFCGTFDPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFRPKKL | SEQ ID NO: 42 |
| C1(R 79D)-C2_s hort2 | CGGILTGESGFIGSEGFPGVYPPNSKCTWKITVPEGKVVVLNFRFIDLESDN LCRYDFVDVYNGHANGQRIGRFCGTFDPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDLSLTADGFIGHYIFR | SEQ ID NO: 43 |

(continued)

| | | |
|---|---|---|
| MBP-C1-C2(L 101A )_sho rt | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDA SLTADGFIGHYIFRPKKL | SEQ ID NO: 44 |
| MBP-C1-C2(L 101A )_sho rt2 | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFRFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDA SLTADGFIGHYIFR | SEQ ID NO: 45 |
| C1-C2(L 101A )_sho rt | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDASLTADGFIGHYIFRPKKL | SEQ ID NO: 46 |

| | | |
|---|---|---|
| C1-C2(L 101A )_sho rt2 | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VVWHIVAPKNQLIELKFEKFDVERDNYCRYDYVAVFNGGEVNDARRIGKYC GDSPPAPIVSERNELLIQFLSDASLTADGFIGHYIFR | SEQ ID NO: 47 |
| MBP-C1(F 44A) C2(L 101A )_sho rt | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFAFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVVWHIVAPKNQLIELKFEKFDVE RDNYCRYDYVAVFNGGEVNDARRIGKYCGDSPPAPIVSERNELLIQFLSDA SLTADGFIGHYIFRPKKL | SEQ ID NO: 48 |
| MBP-C1(F 44A) C2(L 101A )_sho rt2 | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFAFIDLESDNLCRYDFVDVYNGHANGQRIGR FCGTFRPGALVSSGNKMMVQMISDANTAGNGFMAMFSAAEPNERGDQY CGGLLDRPSGSFKTPNWPDRDYPAGVTCVVWHIVAPKNQLIELKFEKFDVE RDNYCRYDYVAVFNGGEVNDARRIGKYCGDSPPAPIVSERNELLIQFLSDA SLTADGFIGHYIFR | SEQ ID NO: 49 |

| | | |
|---|---|---|
| C1(F 44A) C2(L 101A | CGGILTGESGFIGSEGFPGVYPPNSKCTWKITVPEGKVVVLNFAFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDASLTADGFIGHYIFRPKKL | SEQ ID NO: 50 |
| )_sho rt | | |
| C1(R 44A)-C2(L 101A )_sho rt2 | CGGILTGESGFIGSEGFPGVYPPNSKCTWKITVPEGKVVVLNFAFIDLESDN LCRYDFVDVYNGHANGQRIGRFCGTFRPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDASLTADGFIGHYIFR | SEQ ID NO: 51 |
| MBP-C1(R 44A, F78A ,R79 D)-C2(L 101A )_sho rt | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFAFIDLESDNLCRYDFVDVYNGHANGQRIGR FCGTADPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDA SLTADGFIGHYIFRPKKL | SEQ ID NO: 52 |

| | | |
|---|---|---|
| MBP-C1(R 44A, F78A ,R79 D)-C2(L 101A )_sho rt2 | KIEEGKLVIWINGDKGYNGLAEVGKKFEKDTGIKVTVEHPDKLEEKFPQVAA TGDGPDIIFWAHDRFGGYAQSGLLAEITPAAAFQDKLYPFTWDAVRYNGKL IAYPIAVEALSLIYNKDLLPNPPKTWEEIPALDKELKAKGKSALMFNLQEPYF TWPLIAADGGYAFKYAAGKYDIKDVGVDNAGAKAGLTFLVDLIKNKHMNAD TDYSIAEHAFNHGETAMTINGPWAWSNIDTSAVNYGVTVLPTFKGQPSKPF VGVLSAGINAASPNKELAKEFLENYLLTDEGLEAVNKDKPLGAVALKSYEEE LVKDPRVAATMENAQKGEIMPNIPQMSAFWYAVRTAVINAASGRQTVDAA LAAAQTNAAALEVLFQGPASQSPERPVFTCGGILTGESGFIGSEGFPGVYP PNSKCTWKITVPEGKVVVLNFAFIDLESDNLCRYDFVDVYNGHANGQRIGR FCGTADPGALVSSGNKMMVQMISDANTAGNGFMAMFSAAEPNERGDQY CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDA SLTADGFIGHYIFR | SEQ ID NO: 53 |
| C1(R 44A, F78A ,R79 D)-C2(L 101A )_sho rt | CGGILTGESGFIGSEGFPGVYPPNSKCTWKITVPEGKVVVLNFAFIDLESDN LCRYDFVDVYNGHANGQRIGRFCGTADPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDASLTADGFIGHYIFRPKKL | SEQ ID NO: 54 |
| C1(R 44A, F78A ,R79 D)-C2(L 101A )_sho rt2 | CGGILTGESGFIGSEGFPGVYPPNSKCTWKITVPEGKVVVLNFAFIDLESDN LCRYDFVDVYNGHANGQRIGRFCGTADPGALVSSGNKMMVQMISDANTA GNGFMAMFSAAEPNERGDQYCGGLLDRPSGSFKTPNWPDRDYPAGVTC VWHIVAPKNQLIELKFEKFDVERDNYCRYDYVAVFNGGEVNDARRIGKYC GDSPPAPIVSERNELLIQFLSDASLTADGFIGHYIFR | SEQ ID NO: 55 |
| C2-NTR _shor t | CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFRPKKLPTTTEQPVTTTFPVTTGLKPTVALCQQKCRRTGT LEGNYCSSDFVLAGTVITTITRDGSLHATVSIINIYKEGNLAIQQAGKNMSAR LTVVCKQCPLLRRGLNYIIMGQVGEDGRGKIMPNSFIMMFKTKNQKLLDAL KNKQC | SEQ ID NO: 56 |

| | | |
|---|---|---|
| C2-C1-GS | CGGLLDRPSGSFKTPNWPDRDYPAGVTCVWHIVAPKNQLIELKFEKFDVE RDNYCRYDYVAVFNGGEVNDARRIGKYCGDSPPAPIVSERNELLIQFLSDL SLTADGFIGHYIFRGSGSGSGSGSGSGSGSGSGSGSCGGILTGESGFIGSEG FPGVYPPNSKCTWKITVPEGKVVVLNFRFIDLESDNLCRYDFVDVYNGHAN GQRIGRFCGTFRPGALVSSGNKMMVQMISDANTAGNGFMAMFSAA | SEQ ID NO: 57 |
| C1-C2-P1 | CGGILTGESGFIGSEGFPGVYPPNSKCTWKITVPEGKVVVLNFRFIDLESDN LCRYDFVDVYNGHANGQRIGRFCGTFRPGALVSSGNKMMVQMISDANTA GNGFMAMFSAAATSGTEHQFCGGLLDRPSGSFKTPNWPDRDYPAGVTCV WHIVAPKNQLIELKFEKFDVERDNYCRYDYVAVFNGGEVNDARRIGKYCG DSPPAPIVSERNELLIQFLSDLSLTADGFIGHYIFR | SEQ ID NO: 58 |
| C1(D 51H, F78A )-C2(D 52H, N53D )_sho rt | CGGILTGESGFIGSEGFPGVYPPNSKCTWKITVPEGKVVVLNFRFIDLESHN LCRYDFVDVYNGHANGQRIGRFCGTARPGALVSSGNKMMVQMISDANTA GNGFMAMFSAAEPNERGDQYCGGLLDRPSGSFKTPNWPDRDYPAGVTC VWHIVAPKNQLIELKFEKFDVERHDYCRYDYVAVFNGGEVNDARRIGKYC GDSPPAPIVSERNELLIQFLSDLSLTADGFIGHYIFRPKKL | SEQ ID NO: 59 |

All these peptides are novel.

The invention also relates to a nucleic acid molecule containing a nucleic acid sequence coding for a peptide as defined above.

The invention also relates to a vector, linear or circular, comprising the above-mentioned nucleic acid sequence. The vector comprises all the means allowing the transcription into mRNA of the sequence coding the peptides as defined above, such as initiation of transcription domain such as a promoter, STOP codon, and means allowing the translation of the transcribed mRNA.

The skilled person could easily choose the best vector from the ones commercially available for the purpose of expression of the peptides defined above, depending on the chosen expression system (in vitro, in vivo, in bacteria, or eukaryote cells...).

The invention also relates to a cell comprising, either integrated in its genome, or as free nucleic acid molecule, a nucleic acid molecule as defined above, or a vector as defined above.

The invention also relates to a non-human animal comprising a cell, preferably a somatic cell, as defined above.

The invention also relates to a pharmaceutical composition comprising the peptide as defined above, or a nucleic acid molecule as defined above, in association with a pharmaceutically acceptable vehicle, or carrier.

The carrier or vehicle has to be "acceptable" in the sense of being compatible with the other ingredients of the formulation and not deleterious to the recipient thereof. Desirably, the formulations should not include oxidizing agents and other substances with which the peptides are known to be incompatible. The composition may conveniently be presented in unit dosage form and may be prepared by any of the methods well known in the art of pharmacy. All methods include the step of bringing into association the peptide with the carrier or vehicle which constitutes one or more accessory ingredients. In general, the composition is prepared by uniformly and intimately bringing into association the peptide with the carrier or vehicle and then, if necessary, dividing the product into unit dosages thereof.

Examples of suitable pharmaceutical carriers include diluents, solvents, buffers, and/or preservatives. An example of a pharmaceutically acceptable carrier is phosphate buffer that contains NaCl. Other pharmaceutically acceptable carriers include aqueous solutions, non-toxic excipients, salts, preservatives, buffers and the like. Examples of non-aqueous solvents are propylene glycol, polyethylene glycol, vegetable oil and injectable organic esters such as ethyloleate. Aqueous carriers include water, alcoholic/aqueous solutions, saline solutions, parenteral vehicles such as sodium chloride, Ringer's dextrose, etc. Intravenous vehicles include fluid and nutrient replenishers. Preservatives include antimicrobials, anti-oxidants, chelating agents and inert gases. The pH and exact concentration of the various components of the binding composition are adjusted according to routine skills in the art.

The invention also relates to the peptides as defined above, or the pharmaceutical composition as defined above, as medicine or drug.

The medicine or drug according to the invention is formulated for an oral, a parenteral, a topical application in skin or cornea, an intravenous administration to the patient in a need thereof. This drug can be used for animal in a veterinary cure, or for human.

In another aspect, the invention relates to a peptide as defined above, or a pharmaceutical composition as defined above, for its use for the treatment and the prevention of a pathology involving deregulation of a BTP protease.

Since the peptides according to the invention are able to inhibit BTP activity, the peptides according to the invention can be used for preventing or treating pathologies involving a deregulation of these proteases. In particular, the pathologies involving a BTP deregulation are pathologies where at least one BTP is over expressed, or harbors an increased proteolytic activity. Therefore, administration of an efficient dose of a peptide according to the invention could compete with the BTP excess or exacerbated activity, and restore the basal condition, i.e. to treat the associated condition.

In the same way, administration to an animal of an effective amount of the peptide according to the invention can prevent development of symptoms associated with deregulation of a BTP.

The peptides according to the invention may also be used to modulate the deregulation of the activity or expression of BTP substrates, as well as of activators or inhibitors involved in BTP regulation.

Advantageously, the invention relates to the peptide as defined above for its use as defined above, wherein the pathology involving deregulation of BTP proteases is chosen from fibrotic disorders, cancers, metabolic diseases including atherosclerosis, diabetes and nonalcoholic steatohepatitis, and myopathies-, cachexia- or aging-associated muscular troubles.

In another aspect, the invention relates to the use of peptide as defined above, for inhibiting in vitro the activity of a BTP.

For experimental non medical uses, the peptides as defined above can be used for studying the activity and/or function of a BTP. The peptides can then be used at different dosages for modulating the activity of one or more BTP.

Therefore, it is also disclosed a method for in vitro inhibiting a BTP in a sample comprising a step of contacting a BTP with a peptide as defined above.

In one another aspect, the invention relates to a kit comprising a peptide as defined above and a medicine.

In a combined therapy for treating, or preventing, the above-mentioned diseases, it is advantageous to combine the peptide according to the invention with another pharmaceutically effective compound or medicine. This medicine can be any appropriate medicine for treating a pathology involving a BTP deregulation, for instance anti-cancer medicine (small molecules and antibodies), medicine for treating cardiovascular disease (statins, losartan, etc.), anti-fibrotic medicine (pirfenidone, losartan, etc.), etc., could be considered. This list is not limitative, and the skilled person will be able to choose the most appropriate molecule for an association with the peptide according to the invention.

It is also disclosed a method for treating or preventing pathologies involving a deregulation of a BTP protease, preferably the pathologies as listed above, the method comprising administering to an individual in the need thereof, an effective amount of a peptide comprising, consisting essentially, or consisting of the amino acid sequence SEQ ID NO :1, provided that said peptide does not consist in SEQ ID NO: 2 or SEQ ID NO: 3.

Advantageously, it is disclosed the method as defined above, wherein at least one of the amino acids in position 15, 16, 19, 20, 23, 24, 44, 45, 47, 48, 50, 51, 52, 53, 55, 56, 76, 77, 78, 79, 98, 99, 100, 101, 102 and 103 of SEQ ID NO: 1 is substituted by any other amino acid.

Advantageously, it is disclosed the method as defined above, wherein said peptide comprises both amino acid sequences SEQ ID NO: 1 and SEQ ID NO: 4.

Advantageously, it is disclosed the method as defined above, wherein the at least one of the amino acids in position 21, 52, 53, 54, 56, 57, 70, 73, 74, 75, 76, 77, 80, 81, 82, 84, 86, 101, 102, 103, 104 and 106 of SEQ ID NO: 4 is substituted by any other amino acid.

It is also disclosed a peptide comprising, consisting essentially, or consisting of the amino acid sequence SEQ ID NO: 4, provided that said peptide does not consist in SEQ ID NO: 2 or SEQ ID NO: 3.

Advantageously, it is also disclosed the peptide as defined above, to 3, wherein the at least one of the amino acids in position 21, 52, 53, 54, 56, 57, 70, 73, 74, 75, 76, 77, 80, 81, 82, 84, 86, 101, 102, 103, 104 and 106 of SEQ ID NO: 4 is substituted by any other amino acid.

The invention will be better understood in the light of the figures and the following examples.

## Brief description of the drawings

Expression of PCPEs and BTPs in WT and Pcolce2-null (KO) mouse skin

[Fig. 1] represent graphs showing the relative mRNA quantification of Pcolce2 (B) and Pcolce (A) by qRT-PCR. Means $\pm$ SD (n = 6 mice/genotype; Mann-Whitney test for Pcolce; Wilcoxon signed rank test for Pcolce2; *, p < 0.05. Y-axis represents gene expression normalized to *Gapdh.*

[Fig. 2] represents the detection of PCPE-1 by Western blotting (with AF2239 antibody) in skin lysates of WT and Pcolce2 KO mice (12 % acrylamide gel; reducing conditions). Normalization was by total protein amount quantified after Stain-Free detection (means $\pm$ SD; Mann-Whitney test; n = 3 mice/genotype)

[Fig. 3] represents graphs showing relative mRNA quantification of Bmp1-1 (encoding BMP-1), Bmp1-3 (encoding mTLD) and Tll1 mRNA by qRT-PCR (Mann-Whitney test; n = 6 mice/genotype). Y-axis represents gene expression normalized to *Gapdh.*

[Fig. 4] represents TEM pictures of collagen fibrils in the skin of WT and Pcolce2-null (KO) mice (6 week-old). Representative images of n = 8 mice/genotype. Bar = 200 nm.

[Fig. 5] represents transmission electron microscopy pictures of collagen fibrils in WT and Pcolce2-null (KO) mice in skin (A), heart (B) and tail tendon (C) tissue sections. Conditions are the same as in figure 4 for skin but lower magnifications are shown. For heart and tail tendons, images were also obtained from 6 week-old animals. Bars correspond to 1 $\mu$m for heart and 2 $\mu$m for skin and tail tendon. Black arrows indicate collagen fibrils in longitudinal or transverse sections. F, fibroblast; C, cardiomyocyte; BV, lumen of a blood vessel; T, tenocyte.

[Fig. 6] represents graphs showing the distribution of skin collagen fibril diameters for WT and KO mice and median values of all measurements; 3092 fibrils from n = 8 mice were analyzed for each genotype. The graph of the median diameters calculated for individual mice is also shown (means $\pm$ SD; n = 8 mice/genotype; unpaired t-test).

[Fig. 7] represents TEM pictures of the collagen fibrils in heart (A) and tail tendon (B) of 6 week-old WT and KO mice. Representative images of n = 4 mice for each genotype. Bar = 100 nm (heart) or 200 nm (skin, tail tendon)

[Fig. 8] represents graphs showing distributions of collagen fibril diameters for WT and KO mice and median values of all measurements; 807 fibrils from n = 4 mice were analyzed for each genotype in heart and 1855 fibrils from n = 4 mice in tail tendon. The graph of the median diameters calculated for individual mice is also shown (means $\pm$ SD; 4 mice/genotype for heart and tail tendon; unpaired t-test).

[Fig. 9] represents graphs showing the comparison of stretching parameters for the skin of WT and KO mice. Data show means $\pm$ SD of n = 5 or 6 mice for WT and n = 7 mice for KO (age of the mice: 8 weeks; unpaired t-test).

[Fig. 10] represents graphs showing the comparison of the mRNA abundance for the genes encoding PCPE-1 and PCPE-2 by qRT-PCR in normal mouse and human dermal fibroblasts (NMF and NHF respectively). Graphs show means $\pm$ SD (n = 4 mice or 6 human donors; unpaired t-test with Welch's correction: *, p-value < 0.05). Y-axis represents gene expression normalized to *Gapdh.*

[Fig. 11] Immuno-detection of procollagen I, pC-collagen I and C-propeptide of collagen I (see schematic for a definition; PNP = procollagen N-proteinase and PCP = procollagen C-proteinase) with the anti-C-propeptide I (LF-41) antibody in the supernatant of WT and KO skin fibroblasts after 8 h of culture without serum. SDS-PAGE was run in reducing conditions and the same membrane was exposed for 1 min to detect the pro- and pC-collagens and for 3 min to detect the C-propeptide. Quantification of band intensities is shown for 3 mice/condition (means $\pm$ SD; unpaired t-test).

[Fig. 12] Detection of the same processing products as in Fig. 11 in the supernatant of human adult fibroblasts after incubation for 2 h at 37 °C with buffer or 10 nM exogenous BMP-1. When indicated, 750 nM PCPE-1 and/or PCPE-2 were also added to the cleavage assays.

[Fig. 13] Quantification was with n = 7-8 independent experiments (conditions shown in the same order as in (Fig.12)). Means $\pm$ SD are shown and conditions were compared using one-way ANOVA with paired data and Dunnett test for multiple comparisons (*, p-value < 0.05; **, p-value < 0.01).

The effect of PCPE-2 on procollagen C-terminal maturation is concentration-dependent and involves a direct interaction with procollagens

[Fig. 14] Mini-procollagens I, II and III (430 nM) were incubated with 21 nM BMP-1 in the absence or presence of 430 nM PCPE-1 or / and PCPE-2 in a total volume of 30 μl for 1 h. Reaction products were analyzed by SDS-PAGE (4-20 % polyacrylamide gel; non-reducing conditions) and Coomassie Blue staining. Enhancement factors estimated by densitometry are indicated below the gel. Gels are representative of n = 3 independent experiments. (*) indicates the trimeric N-terminal cleavage product of mini-procollagen III (when visible).

[Fig. 15] Cleavage of CPIII-Long (360 nM) by BMP-1 (20 nM) for 1 h in the absence or presence of PCPE-1, PCPE-2, the CUB domains of PCPE-2, the NTR domain of PCPE-2 or combinations of PCPE-1 and PCPE-2 (or its domains). Molar ratios of PCPEs and domains to CPIII-Long are indicated above the gel and enhancement factors are reported below the gel. The gel is representative of n = 3 independent experiments.

[Fig. 16] Quantification of CPIII-Long (360 nM) processing by BMP-1 (16.5 nM) in the presence of increasing amounts of PCPE-1 and PCPE-2 (0-2.2 μM). Analysis was by SDS-PAGE (reducing conditions) on a 15 % acrylamide gel with Sypro Ruby staining and quantification of individual substrate bands.

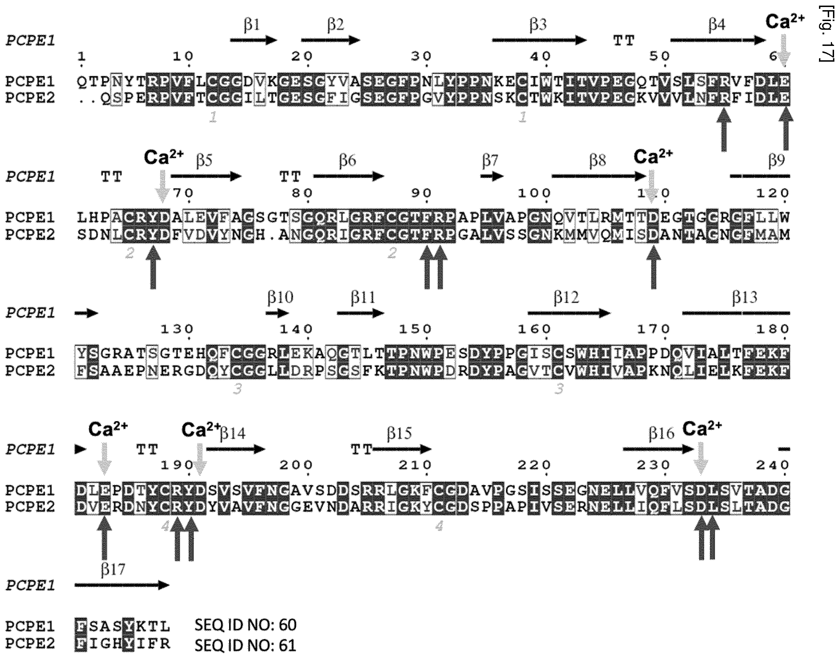

[Fig. 17] Alignment of the CUB domains of human PCPE-1 and PCPE-2 showing the conservation of the residues involved in the interaction with the C-propeptide of procollagen III. Numbering refers to mature human PCPE-1. CUB1: residues 12-123, CUB2: residues 132-248. Identical residues are indicated by white labels on a black background, conservative changes by boxed black on white and non-conservative changes by black on white (identity between the two sequences = 54.3 %). Secondary structures from PDB entry 6FZV. Key residues involved in the interaction of PCPE-1 with procollagen III C-propeptide are indicated with gray arrows. Calcium ligands and cysteines involved in disulphide bridges are also indicated.

[Fig. 18] Superposition of a model of PCPE-2 CUB domains on the X-ray structure (PDB code 6FZV) of the complex between the CUB domains of PCPE-1 and C-propeptide III. Calcium ions are represented as spheres.

[Fig. 19] PCPE-2 binding to immobilized mini-procollagen III (435 RU). Increasing concentrations of PCPE-2 were injected (0.78-50 nM, prepared as serial two-fold dilutions). The best fit of the binding response at equilibrium (steady-state conditions) is also shown (inset). Kinetic constants were outside instrument specifications and could not be determined.

[Fig. 20] PCPE-1 binding to immobilized mini-procollagen III (435 RU). Increasing concentrations of PCPE-1 were injected (0.78-50 nM, prepared as serial two-fold dilutions) and fits were obtained with the kinetic (black dotted lines; heterogeneous ligand) and steady-state (insets) models. The main parameters derived from the best fits obtained in the two conditions are also indicated.

[Fig. 21] Fits of the sensorgrams obtained in the same conditions when PCPE-2 was injected over immobilized C-propeptide III (351 RU). Best fit of the kinetic data was obtained here with the 1:1 binding model.

[Fig. 22] Successive injections of PCPE-2 and PCPE-1 on immobilized mini-procollagen III (435 RU) compared to successive injections of PCPE-2 alone.

[Fig. 23] Successive injections of PCPE-1 + PCPE-2 on immobilized mini-procollagen III (435 RU) compared to the successive injections of PCPE-1 alone.

[Fig. 24] Domain structure of native PCPE-2 and schematic of the PCPE-2 3C construct. The cleavage sequence of the HRV 3C-protease and the position of the insertion site in the linker between the CUB1CUB2 and NTR domains of human PCPE-2 are indicated.

[Fig. 25] SDS-PAGE analysis of the resulting purified proteins in non-reducing conditions. Three μg of purified protein/lane were loaded on the gel. The two bands observed for CUB1CUB2* correspond to distinct O-glycosylated forms.

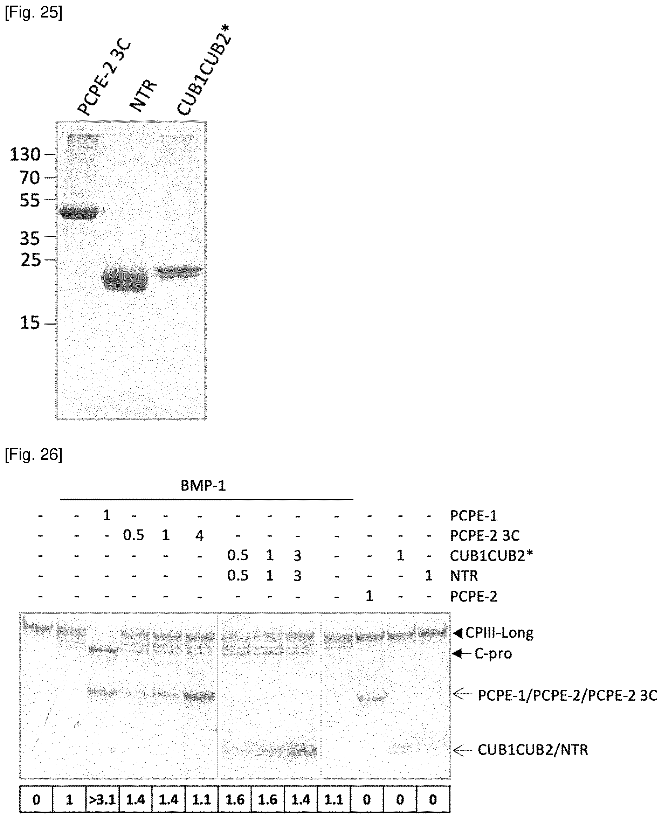

[Fig. 26] PCPE-2 3C and the mixture of CUB1CUB2* and NTR domains have the same effects on BMP-1 PCP activity as PCPE-2 and CUB1CUB2* alone respectively. Cleavage of CPIII-Long (360 nM) by BMP-1 (20 nM) in the absence or presence of PCPE-1, PCPE-2 3C or a mixture of the CUB and NTR domains of PCPE-2 for 1 h. In the absence of BMP-1, CPIII-Long is not affected by PCPE-2 nor its domains (last three lanes). Molar ratios of PCPEs and domains to CPIII-Long are indicated above the gel and the enhancement factors are indicated below the gel. The experiment is representative of n = 3 independent experiments.

[Fig. 27] Interactions of the CUB1CUB2* and NTR domains of PCPE-2 with procollagens. Fits of the sensorgrams obtained with kinetic (black dotted lines; 1:1 binding) or steady-state (inset) models when increasing concentrations of the CUB1CUB2* domains of PCPE-2 were injected (0.78-50 nM, prepared as serial two-fold dilutions) over immobilized mini-procollagen III (435 RU).

[Fig. 28] Interactions of the CUB1CUB2* and NTR domains of PCPE-2 with procollagens. Comparison of the binding of 200 nM of the CUB1CUB2* and NTR domains of PCPE-2 on immobilized mini-procollagen III (435 RU).

PCPE-2 inhibits the cleavage of BMP-1 substrates. Effect of PCPE-1 and -2 on various BMP-1 substrates at 1:1 PCPE:substrate molar ratios (unless otherwise indicated) at 37 °C.

[Fig. 29] The pNa1(V) protein (800 nM) is the monomeric N-terminal region of the $\alpha$1 chain of collagen V and it was incubated with 20 nM BMP-1 for 2 h to monitor the release of the N-terminal TSPN domain (PCPE to pN$\alpha$1(V) molar ratios as indicated above the gel).

[Fig. 30] TSP-1 (200 nM) was incubated with 70 nM BMP-1 for 4 h to generate one N-terminal fragment (45 kDa) and one C-terminal fragment (120 kDa).

[Fig. 31] The ectodomain of betaglycan (780 nM) is cleaved by BMP-1 (50 nM) in two main products (N-ter, C-ter) in the conditions of the assay (4 h).

[Fig. 32] Chordin (present in two forms in the starting material, resulting from alternative splicing according to the manufacturer; 370 nM) yielded 3 products (referred to as N-ter, internal and C-ter) when incubated with 20 nM BMP-1 for 4 h.

[Fig. 33] Endorepellin (265 nM) corresponds to the C-terminal part of perlecan and was cleaved by BMP-1 (18 nM) in two fragments (LG1-2 and LG3) after 1 h.

[Fig. 34] The ectodomain of LDLR (390 nM) was converted to a shorter fragment (C-ter) in the presence of 1.5 nM BMP-1 for 1 h. In Fig.29 to 33 detection was by SDS-PAGE (reducing conditions) with Coomassie Blue staining except for endorepellin and LDLR which were detected with Sypro Ruby staining. (*) indicates BMP-1 position when visible.

[Fig. 35] Immuno-detection of endogenous LDLR in 293-EBNA cell lysates, after stable transfection with an empty vector or the same vector containing sizzled sequence or PCPE-2 sequence. Expression of sizzled and PCPE-2 was also evidenced by immunoblotting with anti-His antibody in the supernatant of transfected cells.

The CUB domains of PCPE-2 are responsible for its inhibitory activity and the non-catalytic domains of BMP-1 are required for inhibition.

[Fig. 36] Quantification of BMP-1 activity on the fluorogenic peptide Mca-YVADAPK(Dnp)-OH in the absence or presence of increasing concentrations of PCPE-2, PCPE-2 3C, the CUB1CUB2* domains of PCPE-2, the NTR domain of PCPE-2 or PCPE-1, at the indicated molar ratios to BMP-1 (7 nM). Means $\pm$ SD of n = 3 independent experiments run in duplicate.

[Fig. 37] The apparent inhibition constant (Kiapp) of PCPE-2 was measured in the same conditions with PCPE-2 concentrations between 0.07 and 690 nM and calculated using the Morrison equation. Means $\pm$ SD of n = 3 independent experiments run in duplicate.

[Fig. 38] Effect of PCPE-2 on the cleavage of the fluorogenic peptide by the catalytic domain of BMP-1 (BMP-1cat, 8 nM). Means $\pm$ SD of n = 3 independent experiments run in duplicate.

[Fig. 39] Comparison of the binding of 50 nM of PCPE-1, PCPE-2 and its domains (CUB1CUB2* and NTR) on immobilized BMP-1 (974 RU).

[Fig. 40] Comparison of the binding of 50 nM BMP-1 and BMP-1cat on immobilized PCPE-2 (646 RU).

[Fig. 41] Fits of sensorgrams obtained with the kinetic (black dotted lines; heterogeneous ligand) or steady-state (inset) model when increasing concentrations of PCPE-2 (1.56-100 nM, prepared as serial two-fold dilutions) were injected over immobilized BMP-1 (974 RU).

[Fig. 42] Same as in Figure 42 for the CUB1CUB2* domains of PCPE-2.

Interactions of PCPE-2 with procollagen and BMP-1 are mutually exclusive.

[Fig. 43] Competition experiments consisting of an injection of 200 nM PCPE-2 for 60 s followed by an injection of PCPE-2 (200 nM) alone or in combination with 50, 100 or 200 nM BMP-1 for another 60s. The immobilized protein was C-propeptide III (351 RU).

[Fig. 44] Same as in Fig. 43 with PCPE-1 instead of PCPE-2.

Effect of PCPE-2 on mTLL-1 and meprins.

[Fig. 45] SDS-PAGE analysis in non-reducing conditions of the cleavage of CPIII-Long (320 nM) by mTLL-1 (17 nM) in the absence or presence of PCPE-1 or PCPE-2 for 4 h. Molar ratios of PCPEs to CPIII-Long are indicated above the gel and the enhancement factors are indicated below the gel. The gel is representative of n = 3 independent experiments.

[Fig. 46] Comparison of mTLL-1 (50 nM) and BMP-1 (10 nM) activities on the fluorogenic peptide Mca-YVADAPK(Dnp)-OH, in the absence or presence of PCPE-1 or PCPE-2 (concentrations corresponding to indicated molar ratios to protease). Means $\pm$ SD (n = 3 independent experiments run in duplicate).

[Fig. 47] SDS-PAGE analysis in non-reducing conditions of the cleavage of CPIII-Long (470 nM) by meprin $\alpha$ (3 nM) or meprin $\beta$ (1.5 nM), in the absence or presence of equimolar PCPE-1 or PCPE-2 concentrations (470 nM), for 15 min. (*) indicates the N-terminal cleavage product of PCPE-1 by meprins when visible.

[Fig. 48] Comparison of BMP-1 (10 nM), meprin $\alpha$ (1 nM) or meprin $\beta$ (0.5 nM) activities on the fluorogenic peptide Mca-YVADAPK(Dnp)-OH, in the absence or presence of PCPE-1 or PCPE-2. Means $\pm$ SD (n = 3-5 independent experiments run in duplicate).

[Fig. 49] The cleavage of CD99 by meprins is not affected by PCPE-1 nor PCPE-2. The recombinant ectodomain of human CD99 (fused to the Fc region of human IgG; 500 nM) was incubated alone or with 1 nM meprin $\alpha$ or 0.33 nM meprin $\beta$ for 30 min to generate one or several fragment(s). When present, PCPE-1 and PCPE-2 were added at the same concentration as the substrate (500 nM; molar ratio to protease $\geq$ 500). Detection was by SDS-PAGE with Sypro Ruby staining. (*) indicates PCPE-1 cleavage products generated by meprins.

[Fig. 50] SDS-PAGE analysis of purified PCPE-2 protein in reducing (+ DTT) or non-reducing (- DTT) conditions (7 $\mu$g of protein/lane).

[Fig. 51] Immunodetection of PCPE-1 and PCPE-2 in 250 ng of each recombinant protein using the AF2627 antibody to detect PCPE-1 and the home-made antibody to detect PCPE-2 (10 % acrylamide gel; reducing conditions).

Overview of BTP regulation by PCPE-1 and PCPE-2. (hereafter C1-C2 = CUB1CUB2)

[Fig. 52] PCPE-1 has no effect on the BTP-dependent cleavage of non-collagenous substrates while PCPE-2 acts as a direct and potent inhibitor of these cleavages.

[Fig. 53] In the case of fibrillar procollagens, PCPE-1 enhances the C-terminal maturation of procollagens I-III through the binding of CUB1CUB2 to C-propeptides. PCPE-2 binds procollagens and BMP-1 with similar affinities but cannot interact with both partners simultaneously, thereby precluding the efficient processing of procollagens. The net effect (enhancement, no effect, inhibition) therefore depends on the relative concentrations of the three partners (procollagen, protease, PCPE-2).

[Fig. 54] Schematic representation of the peptides according to the invention.

[Fig. 55] Effect of PCPE-2 on procollagen C-terminal maturation. (A) Cleavage of a model procollagen III substrate (CPIII-Long; 360 nM) by BMP-1 (20 nM) for 1 h in the absence or presence of PCPE-2 and C1-C2*. Molar ratios of PCPE-2 and C1-C2* to CPIII-Long are indicated in the table above the gel and enhancement factors are reported below the gel. (B) Quantification of CPIII-Long (360 nM) processing by BMP-1 (16.5 nM) in the presence of increasing amounts of PCPE-1 and PCPE-2 (0-2.2 $\mu$M).

[Fig. 56] PCPE-2 inhibits the cleavage of non-collagenous BTP substrates. (A-C) Effect of PCPE-2 on various non-collagenous BTP substrates at 1:1 PCPE-2:substrate molar ratios. (A) TSP-1 (200 nM) was incubated with 70 nM BMP-1 for 4 h to generate one N-terminal fragment (45 kDa) and one C-terminal fragment (120 kDa). (B) The ectodomain of betaglycan (780 nM) is cleaved by BMP-1 (50 nM) in two main products (N-ter, C-ter) in the conditions of the assay (4 h). (C) The ectodomain of LDLR (390 nM) was converted to a shorter fragment (C-ter) in the presence of 1.5 nM BMP-1 for 1 h.

[Fig. 57] Purity of recombinant human PCPE-2 and strategy to obtain C1-C2 from MBP-C1-C2. (A) SDS-PAGE analysis of purified PCPE-2 protein. (B) Immunodetection of PCPE-1 in 250 ng of PCPE-2 and PCPE-1. (C) SDS-PAGE analysis of purified PCPE-2-3C, MBP-C1-C2, C1-C2* and C1-C2. The two bands observed for C1-C2* and C1-C2 correspond to distinct O-glycosylated forms. (D) Strategy to obtain the C1-C2 and C1-C2* templates from PCPE-2-3C and MBP-C1-C2 constructs.

PCPE-2 is a potent inhibitor and binder of the BTPs.

[Fig. 58] The apparent inhibition constant (Kiapp) of PCPE-2 was measured with the fluorogenic peptide Mca-YVADAPK(Dnp)-OH with 7 nM BMP-1 and PCPE-2 concentrations between 0.07 and 690 nM.

[Fig. 59] Determination of the dissociation constant of the PCPE-2/BMP-1 complex using the steady-state fit of the response measured by surface plasmon resonance when increasing concentrations of PCPE-2 (1.56-100 nM, prepared as serial two-fold dilutions) were injected over immobilized BMP-1 (974 RU).

[Fig. 60] mTLL-1 is also inhibited by PCPE-2 and inhibition of BMP-1 by PCPE-2 is recapitulated by the CUB domains of PCPE-2 (C1-C2*) which are sufficient.

[Fig. 61] Determination of the dissociation constant of the C1-C2*/BMP-1 complex using the steady-state fit of the response measured by surface plasmon resonance when increasing concentrations of C1-C2* (1.56-100 nM, prepared as serial two-fold dilutions) were injected over immobilized BMP-1 (974 RU).

[Fig. 62] Determination of the dissociation constant of the C1/BMP-1 complex using the steady-state fit of the response measured by surface plasmon resonance when increasing concentrations of C1 (50-3000 nM, prepared as serial two-fold dilutions) were injected over immobilized BMP-1 (1492 RU).

[Fig. 63] Determination of the dissociation constant of the C2NTR/BMP-1 complex using the steady-state fit of the response measured by surface plasmon resonance when increasing concentrations of C2NTR (3.9-250 nM, prepared as serial two-fold dilutions) were injected over immobilized BMP-1 (974 RU).

[Fig. 64] PCPE-2 is a specific inhibitor of the BTPs, not affecting other proteases from the astacin subgroup. Comparison of BMP-1 (10 nM), meprin $\alpha$ (1 nM) or meprin $\beta$ (0.5 nM) activities on the fluorogenic peptide Mca-YVADAPK(Dnp)-OH, in the absence or presence of PCPE-2.

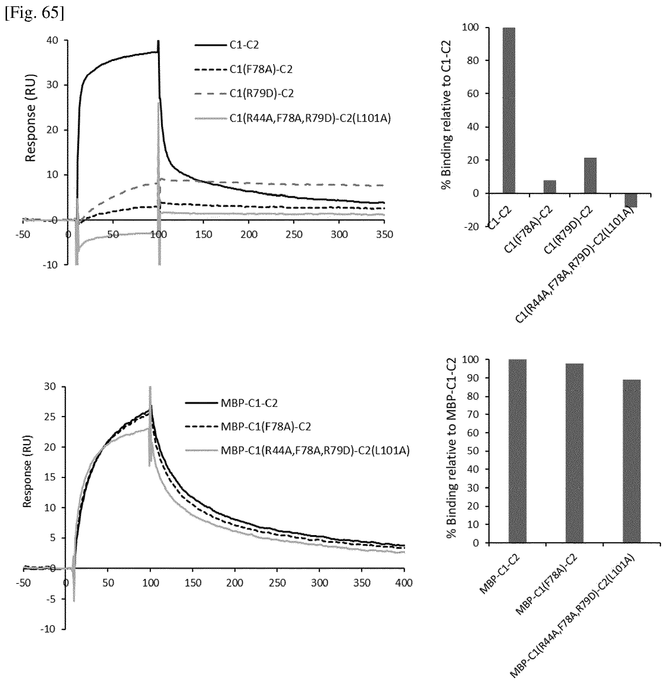

[Fig. 65] Mutations of the CUB domains of PCPE-2 abolish its ability to interact with procollagens without affecting its binding to BMP-1. (upper panels) Comparison by surface plasmon resonance of the binding of 50 nM of C1-C2 and its mutants C1(F78A)-C2, C1(R97D)-C2 and C1(R44A,F78A,R97D)-C2(L101A) on immobilized mini-procollagen III (437 RU) and of the responses measured 5s before the end of injection. (lower panels) Comparison by surface plasmon resonance of the binding of 50 nM of MBP-C1-C2 and its mutants MBP-C1(F78A)-C2, MBP-C1(R97D)-C2 and MBP-C1(R44A,F78A,R97D)-C2(L101A) on immobilized mini-procollagen III (1302 RU) and of the responses measured 5 s before the end of injection.

[Fig. 66] Mutations of the CUB domains of PCPE-2 abolish its ability to activate procollagen C-terminal maturation but not its inhibitory potency towards BTPs. (A) Cleavage of a model procollagen III substrate (Mini-III; 430 nM) by BMP-1 (10 nM) for 1 h in the absence or presence of 430 nM C1-C2, C1(F78A)-C2 and C1(R44A,F78A,R97D)-C2(L101A). (B) BMP-1 is still efficiently inhibited by C1(F78A)-C2 mutant in a fluorogenic peptide cleavage assay.

## Examples

**Example 1:** Identification of PCPE-2 as the first endogenous specific inhibitor of human BMP-1/tolloid-like proteinases

The inventors have taken a closer look at the role of PCPE-2 in collagen maturation and assembly, both in vivo and in vitro. The inventors found that PCPE-2, in contrast to PCPE-1, has no significant effect on collagen organization and processing in Pcolce2-null mice. Unexpectedly however, it has the unique ability to form a tight complex with BMP-1 and to inhibit its proteolytic activity. This specific feature endows PCPE-2 with new possibilities to fine-tune BTP activities and identifies PCPE-2 as the long-awaited endogenous specific inhibitor of mammalian BTPs.

RESULTS

Pcolce2-null mice do not show any major defects in collagen fibrils and PCPE-2 does not stimulate the procollagen I processing activity of BMP-1 in fibroblast medium

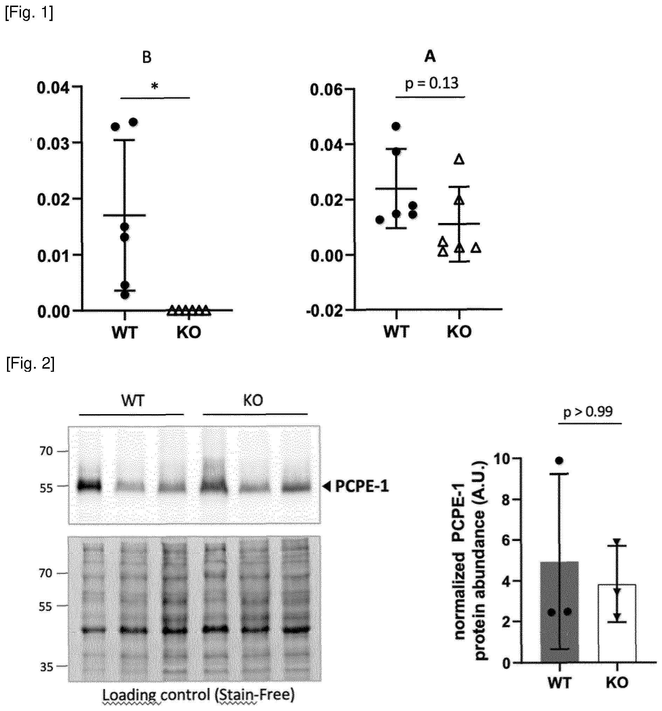

Pcolce2-null mice are viable and fertile and do not show any obvious phenotype in the absence of challenge. In preliminary experiments, it was checked that there was no compensation of the absence of PCPE-2 by PCPE-1 in these mice and that the expression of the BTPs involved in procollagen maturation was not affected by the lack of PCPE-2 (**Figures 1-3**). Then, the inventors took advantage of this model to determine if PCPE-2 was important for the homeostasis of tissues rich in collagen I, as is the case for PCPE-1.

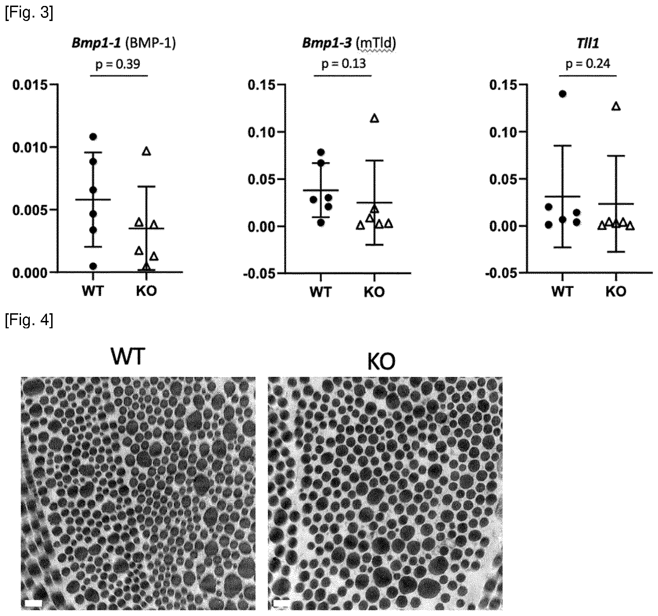

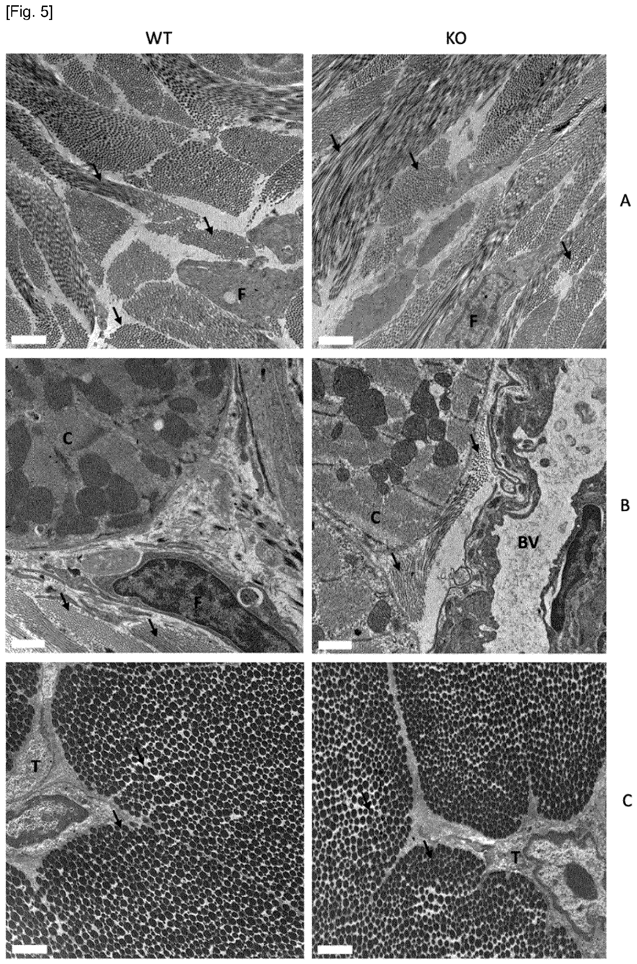

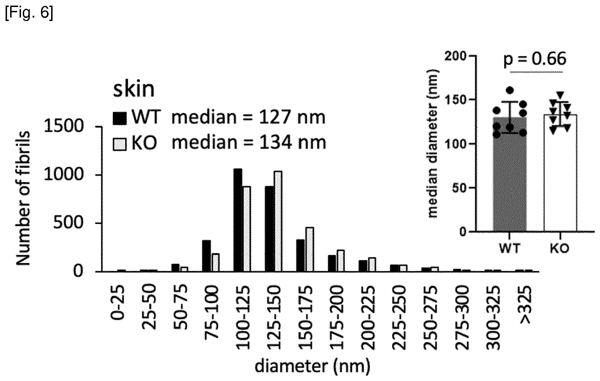

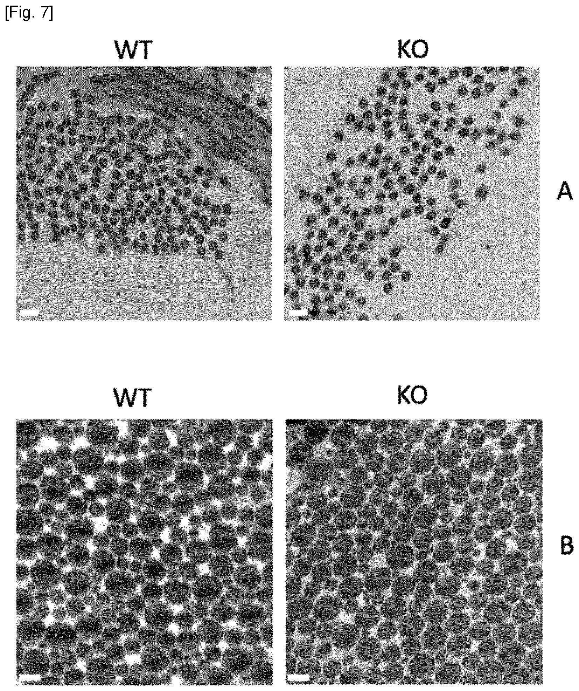

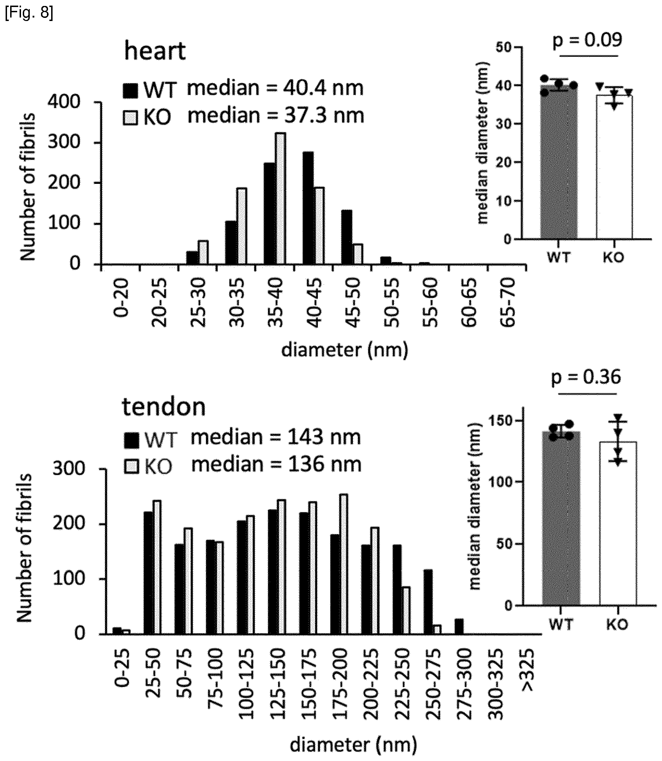

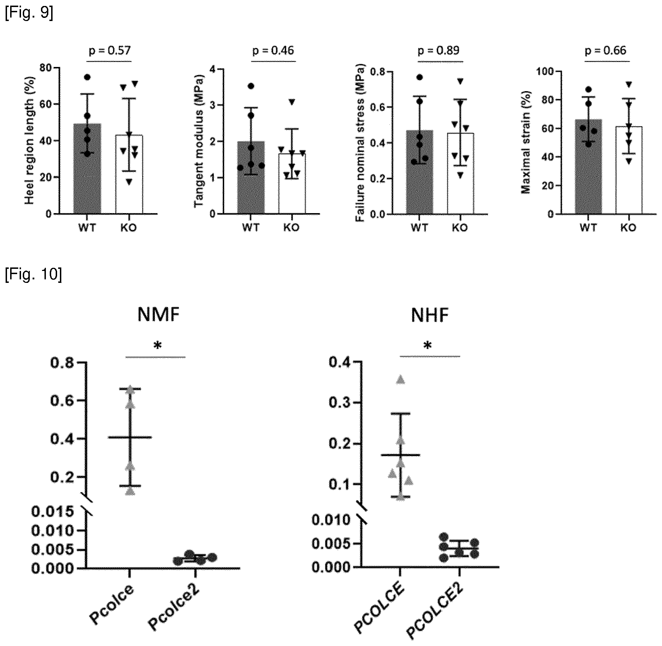

First, the inventors analyzed the organization and morphology of collagen fibrils in the skin of wild-type (WT) and Pcolce2-null (KO) mice by transmission electron microscopy (TEM). In both genotypes, fibroblasts were surrounded by intertwined bundles of oriented collagen fibrils (**Figure 4** and **Figure 5**), which are typical of skin, and there was no sign of fibril fusion or irregular shape. The inventors also measured the diameter of more than 3,000 fibrils in TEM pictures of WT and KO skin and found that the median of the distribution was shifted by no more than 5 % and that the difference between the two genotypes was not significant when median diameters were compared for individual mice (**Figures 6).** Similar results were obtained in the heart, despite the fact that it is the major site of Pcolce2 expression, and in tendons, where 100 % of collagen fibrils were previously found to present irregular (scalloped) shapes in the absence of PCPE-1 (**Figure 5, Figures 7 and 8**). Finally, uniaxial traction assays were also performed to determine if the absence of PCPE-2 had any impact on skin mechanical properties. The tangent modulus, heel region length, failure stretch and ultimate tensile stress were measured for WT and KO mice (**Figure 9**) but none of these parameters differed significantly between the two types of mice, indicating that PCPE-2 does not play a major role in the control of skin collagen organization and mechanical properties.

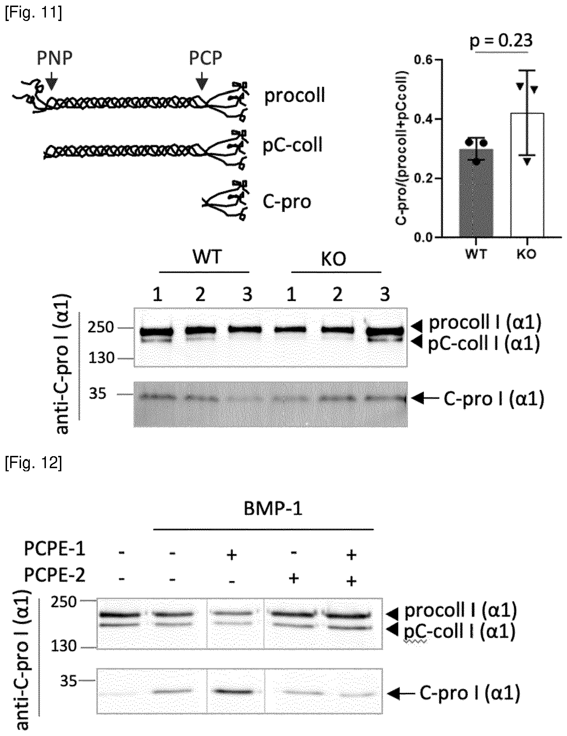

To get further insights into the effect of PCPE-2 on procollagen maturation, fibroblasts from mouse and human skins were extracted and the relative levels of mRNA present in these cells were compared for PCPE-1 and PCPE-2. Interestingly, the mean PCOLCE gene expression was more than 40-fold higher than the mean PCOLCE2 gene expression in fibroblasts from both species (**Figure 10**), confirming the previous result obtained at the protein level by Xu and colleagues who found that PCPE-1 was much more abundant than PCPE-2 in human fibroblast cell extracts. The inventors then analyzed procollagen I processing in the conditioned medium of WT and Pcolce2-null fibroblasts 8 h after serum starvation. In contrast to what has been previously described for Pcolce-null mouse embryonic fibroblasts (MEFs) and corneal keratocytes but in agreement with the low abundance of PCPE-2 in dermal fibroblasts, there was no delay in the C-terminal maturation of procollagen I, as revealed by similar ratios of C-propeptide I to uncleaved precursors (procollagen I and pC-collagen I) in mouse fibroblast supernatants of both genotypes (**Figure 11**).

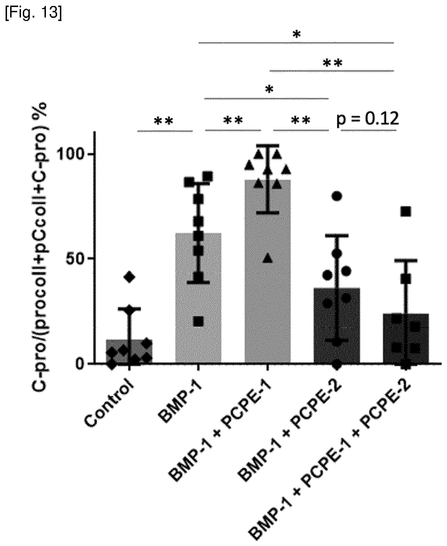

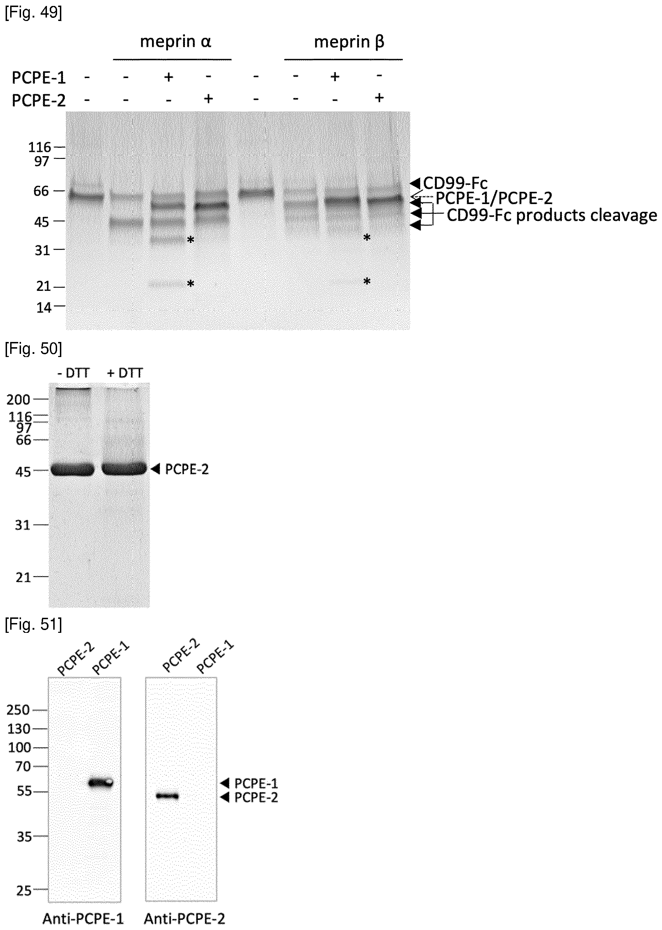

To get a better understanding of its activity, the inventors also produced recombinant PCPE-2 fused to a 6 Histidine-tag at the N-terminus, in 293-T cells, and purified the protein by successive steps of heparin sepharose and Ni-NTA agarose chromatographies. The resulting protein was more than 95 % pure (**Figure 50**) and was not contaminated by endogenous PCPE-1, as confirmed by immunoblotting (**Figure 51**). Recombinant PCPE-2 was then incubated with the supernatant of human adult fibroblasts in the presence of BMP-1 and its PCP enhancing activity on procollagen I was compared to that of recombinant PCPE-1 in the same conditions. While PCPE-1 addition led to a significant increase in C-propeptide release when compared to the condition with BMP-1 alone, PCPE-2 was unable to enhance BMP-1 PCP activity and even displayed inhibitory activity (**Figures 12 and 13**). Even more surprising, PCPE-2 seemed to counteract the activity of PCPE-1 as the simultaneous addition of equimolar amounts of PCPE-1 and -2 failed to reproduce the enhancement obtained by PCPE-1 alone (**Figures 12 and 13**).

PCPE-2 efficiently binds procollagens but does not behave like a canonical procollagen C-proteinase enhancer

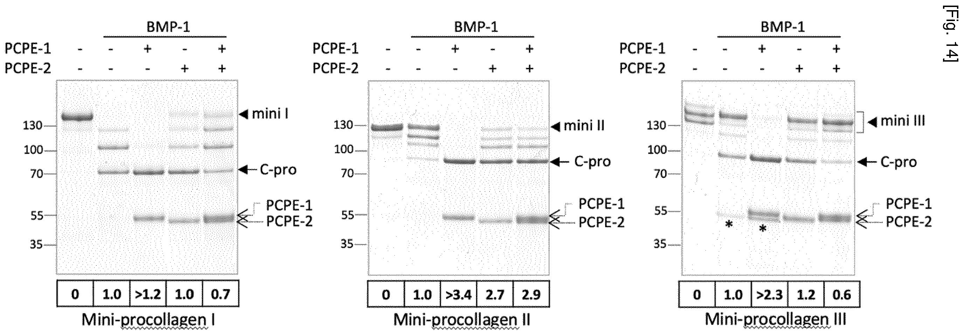

To get further insight into this unexpected behavior of PCPE-2, the inventors ran a series of in vitro experiments with three different mini-procollagens corresponding to truncated versions of fibrillar procollagens I, II and III, lacking the N-propeptides and made of short triple helices and intact C-telopeptides and C-propeptides. These mini-procollagens were cleaved by BMP-1 and their cleavages were efficiently stimulated by PCPE-1 when the latter was present in equimolar amounts with the substrate (**Figure 14**). Interestingly, when tested in the same conditions, the enhancing effect of PCPE-2 was hardly detectable with mini-procollagens I and III but was more clearly evidenced with mini-procollagen II (**Figure 14**). The enhancement factor, calculated from band intensities, as described in Materials and Methods, remained however substantially lower in all cases than with PCPE-1 which allowed 100 % of processing for all mini-procollagens in the conditions of the assay.

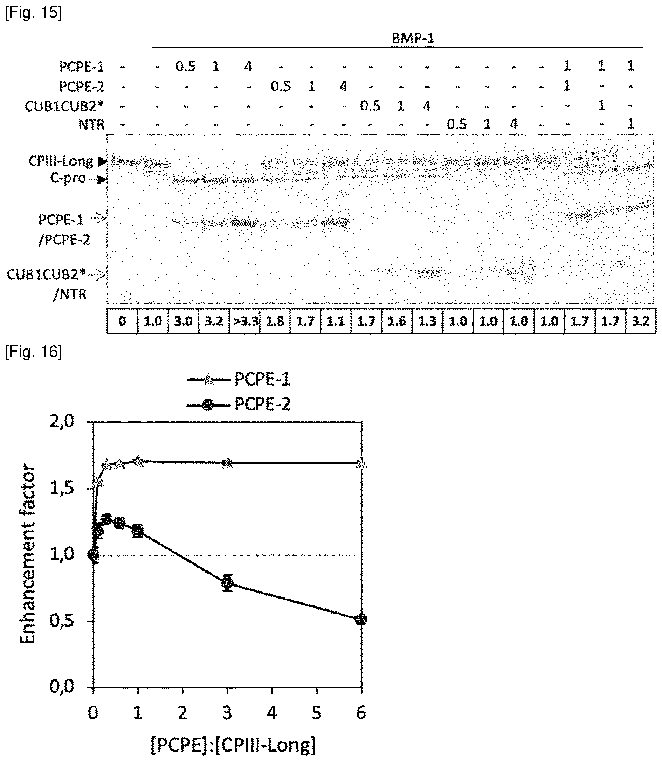

Since the enhancing activity of PCPE-1 is known to depend on its molar ratio to procollagen, the inventors next tried to vary the concentrations of PCPE-1 and PCPE-2 in the assay. To achieve this, the inventors used another model substrate derived from procollagen III, called CPIII-Long, and previously found to be well-suited for the precise quantification of BMP-1 C-proteinase activity. Whereas the enhancing activity of PCPE-1 reached a plateau between 0.5 and 1 equivalent (PCPE:CPIII-Long trimer molar ratio) and remained stable at the maximum cleavage rate with 4 equivalents (enhancement factor > 3), PCPE-2 activity also reached a plateau between 0.5 and 1 equivalent, at the relatively high enhancement factor of 1.7-1.8, but then decreased to the basal level defined by BMP-1 alone at 4 equivalents (**Figure 15**). Using an even larger range of PCPE:CPIII-Long ratios (from 0:1 to 6:1), the inventors actually found that the effect of PCPE-2 on BMP-1 procollagen-processing activity followed a bell-shaped curve (**Figure 16**), with activation at lower concentrations (ratios from 0.1:1 to 1:1) and inhibition at higher concentrations (ratios $\geq$ 3:1). In the conditions of this experiment, the maximum enhancement factor with PCPE-2 was 1.3-fold and the maximum inhibition reached 50 % at the 6:1 ratio while PCPE-1 activity remained stable at the enhancement factor of 1.7 for all ratios superior to 0.3:1. This unexpected result indicated that PCPE-2 did not behave like the canonical PCPE-1 enhancer and that the enhancing activity of PCPE-2 was hindered by another process that was potent enough to abrogate BMP-1 stimulation and could even lead to the inhibition of procollagen cleavage.

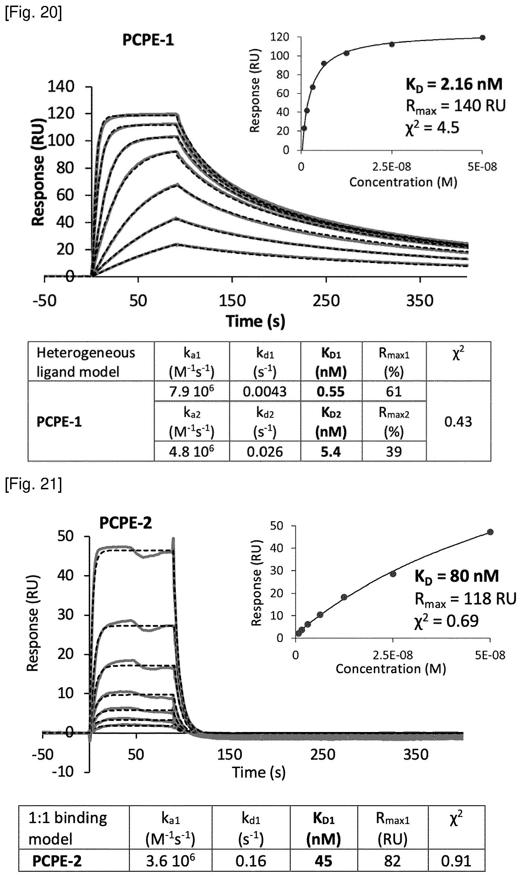

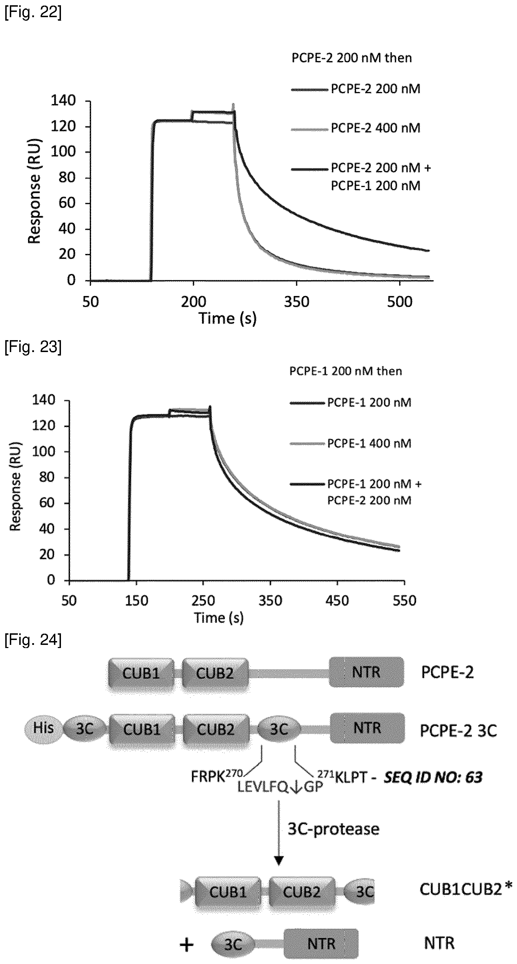

Since the inventors know from previous studies that the interaction of the CUB domains of PCPE-1 with procollagens is the main driver of its enhancing activity, the inventors then looked if PCPE-2 could also bind procollagens. First, the inventors observed that the PCPE-1 residues that were previously shown to be involved in the interaction with procollagen III were perfectly conserved in PCPE-2 (**Figure 17**). In addition, when a model of the CUB domains of PCPE-2 was superimposed on the structure of the complex between the C-propeptide of collagen III and the CUB domains of PCPE-134 (**Figure 18**), the two PCPEs seemed to adopt a similar conformation with a rmsd (root-mean-square deviation) between the atomic positions of alpha carbons in CUB domains of 3.25 Å. To confirm the binding of PCPE-2 to procollagens, the inventors analyzed the interaction of recombinant PCPE-2 with immobilized mini-procollagen III by surface plasmon resonance (SPR). The inventors observed that PCPE-2 also interacted efficiently with mini-procollagen III (**Figure 19**), leading to a dissociation constant of 11 nM (inset in **Figure 19**) to be compared to 2.2 nM for PCPE-1 (**Figure 20**), indicating that PCPE-1 leads to a slightly more stable complex with mini-procollagen III than PCPE-2. Like for PCPE-1, the main site of interaction of PCPE-2 on mini-procollagen III was found to be the C-propeptide and interaction with this region alone led to a dissociation constant of 45 nM (**Figure 21**). Furthermore, the inventors could demonstrate that both proteins bound to overlapping sites on mini-procollagen III as there was no substantial increase in the recorded SPR signal when PCPE-1 was injected on a mini-procollagen III surface which was already saturated with PCPE-2 or vice-versa (**Figure 22, Figure 23**).

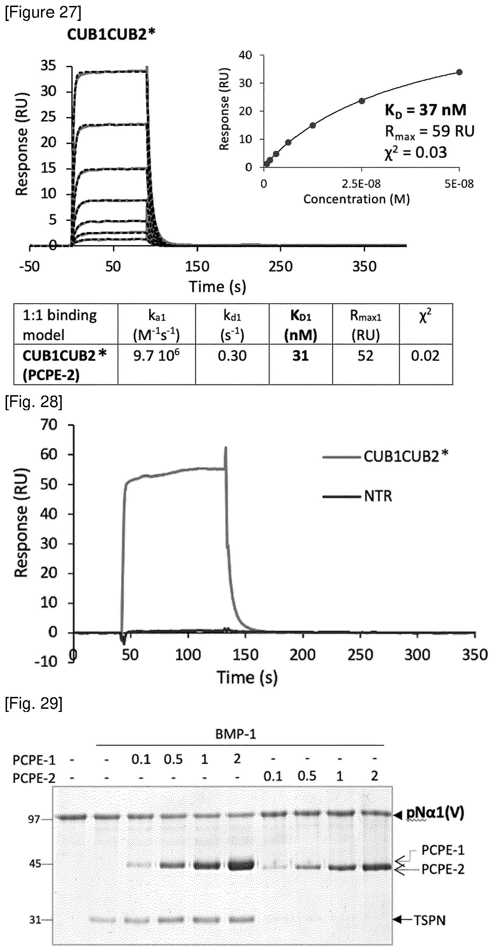

In order to identify the domains of PCPE-2 involved in its interaction with procollagens, the inventors also produced the CUB and NTR domains of PCPE-2 separately, using a PCPE-2 construct containing an internal HRV 3C-protease cleavage site (named PCPE-2 3C hereafter) **(Figures 24 and 25).** After purification, these domains were compared to full-length PCPE-2 and PCPE-2 3C in a CPIII-Long cleavage assay (**Figure 15** and **Figure 26**). Interestingly, while the NTR domain had no impact on substrate processing, PCPE-2 3C and the derived CUB1CUB2* domains could fully recapitulate the activity of full-length PCPE-2. No further change in CUB1CUB2* activity was observed when the NTR domain was also present **(Figure 26)**. In agreement with the observation that the CUB domains of PCPE-2 played the most important role, these bound to mini-procollagen III with a KD around 30 nM (**Figure 27**) while no interaction between the NTR domain and mini-procollagen III was found (**Figure 28**). This suggests that the CUB domains are responsible for PCPE-2 activity, similarly to what was previously described for PCPE-1. Also, the inventors can conclude that the interaction of PCPE-2 with procollagens alone is unlikely to explain the complex activity profile described in **Figure 16** as the main function-related features of PCPE-1 are conserved in PCPE-2.

PCPE-2 is a potent inhibitor of BMP-1

In addition to its PCP enhancing activity, PCPE-1 is also known to stimulate the cleavage of the N-terminal (TSPN) domain of the α1 chain of procollagen V by BMP-1, another activity related to collagen fibril assembly. To determine if PCPE-2 could play a similar role, the inventors monitored the release of the TSPN domain from a truncated form of the procollagen V N-propeptide, called pNα1 (V), by SDS-PAGE. As expected, more TSPN was detected when PCPE-1 was present (at molar ratios to pNα1(V) between 0.1:1 and 2:1) than when BMP-1 alone was incubated with the substrate (**Figure 29**). In contrast, there was no visible TSPN product when PCPE-2 was added at all the tested molar ratios, suggesting that PCPE-2 strongly inhibited BMP-1 activity on pNα1(V).

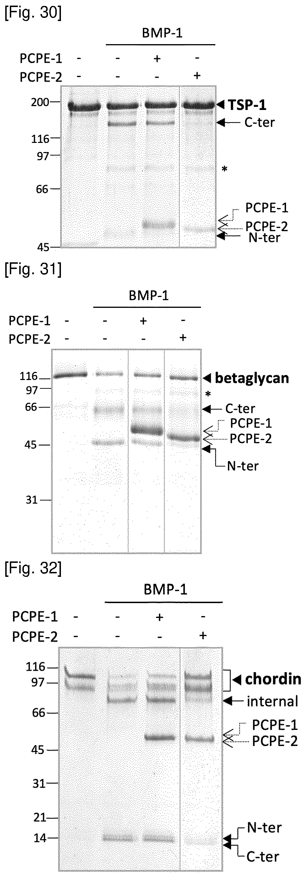

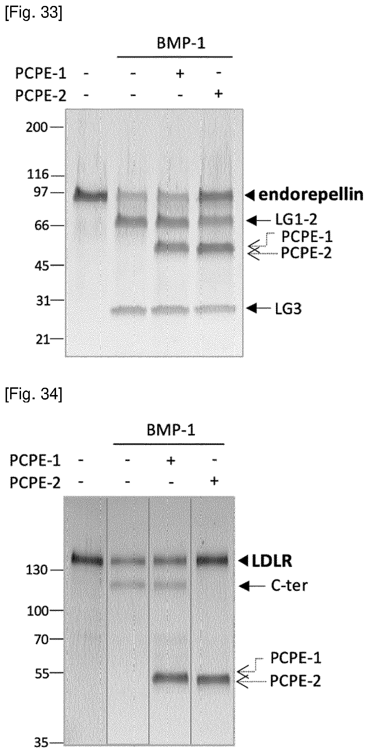

Even more surprisingly, the inventors observed that PCPE-2 also reduced the processing of several other known BMP-1 substrates on which PCPE-1 had no effect. This was the case for thrombospondin-1 (TSP-1), a recently described matricellular substrate involved in the control of cell adhesion and TGF-β activation. Its cleavage products became hardly visible when PCPE-2 was added to the assay at a 1:1 molar ratio (**Figure 30**). Similarly, the processing of betaglycan, another BMP-1 substrate acting both as a TGF-β receptor (TGF-β RIII) at the cell surface and as a TGF-β antagonist when it is released in the extracellular environment, was reduced in the presence of PCPE-2 (**Figure 31**). PCPE-2 also inhibited the cleavage of chordin, a major antagonist of the BMP-2 and BMP-4 growth factors, and of endorepellin, an angiostatic fragment of perlecan (**Figures 32 and 33**). Finally, the BMP-1-dependent cleavage of LDLR, a receptor that mediates the endocytosis of LDL particles, was completely blocked by PCPE-2 (**Figure 34**). In summary, PCPE-2 inhibited the cleavages of all the BMP-1 non-collagenous substrates that the inventors were able to test, suggesting that it behaves as a broad-range inhibitor of BMP-1.

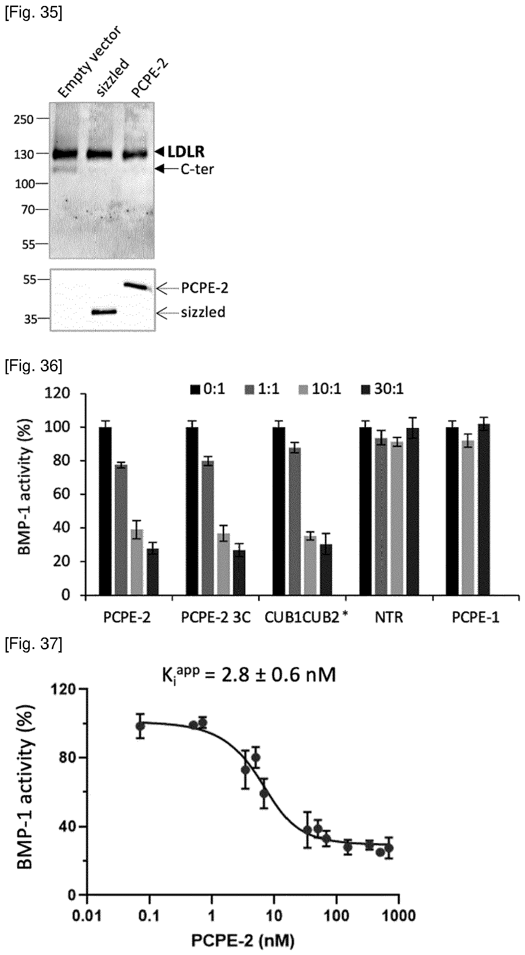

In order to check that this newly observed inhibitory activity of PCPE-2 was not an artifact of the protocol used to purify the recombinant protein, the inventors looked at the processing of an endogenous substrate naturally expressed in 293-EBNA cells. Among the various possible substrates that the inventors tested, the C-terminal fragment of LDLR generated by BMP-1 could be readily detected in the cell lysate of 293-EBNA cells. The dependence on endogenous BMP-1 was confirmed by the comparison of three 293-cell lines stably expressing either PCPE-2, the corresponding empty vector or the Xenopus protein sizzled (not present in mammals) which is known to be a potent and specific exogenous inhibitor of human BTPs. The inventors found that LDLR C-terminal proteolytic product was clearly diminished in PCPE-2- and sizzled-transfected cells compared to cells transfected with the corresponding empty vector (**Figure 35**), confirming PCPE-2 inhibitory function in a cell-based assay.

Finally, the regulation of BMP-1 activity by PCPE-2 was assessed using the fluorogenic peptide Mca-YVADAPK(Dnp)-OH that can be cleaved by BMP-1 between the first alanine and the aspartate. Whereas the addition of PCPE-1 did not modify the cleavage rate of the peptide up to a 30:1 PCPE-1:BMP-1 molar ratio, PCPE-2 was also found to inhibit this cleavage at all tested ratios (from 1:1 to 30:1; **Figure 36**). The apparent inhibition constant (Kiapp) value for PCPE-2 was calculated to be $2.8 \pm 0.6$ nM (**Figure 37**) which indicated that PCPE-2 was a tight-binding inhibitor of BMP-1. However, it failed to decrease BMP-1 activity down to zero with around 25 % residual activity at the highest PCPE-2 concentration (690 nM). This result suggested that PCPE-2 was not a competitive inhibitor directly binding to BMP-1 active site.

The inhibitory activity of PCPE-2 is borne by its CUB domains and requires BMP-1 non-catalytic domains

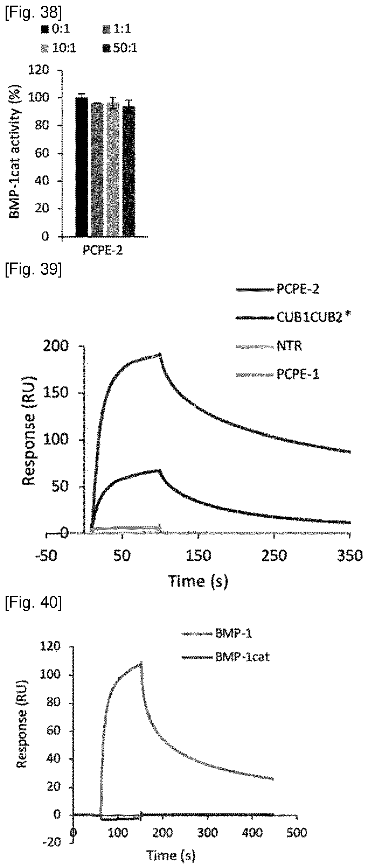

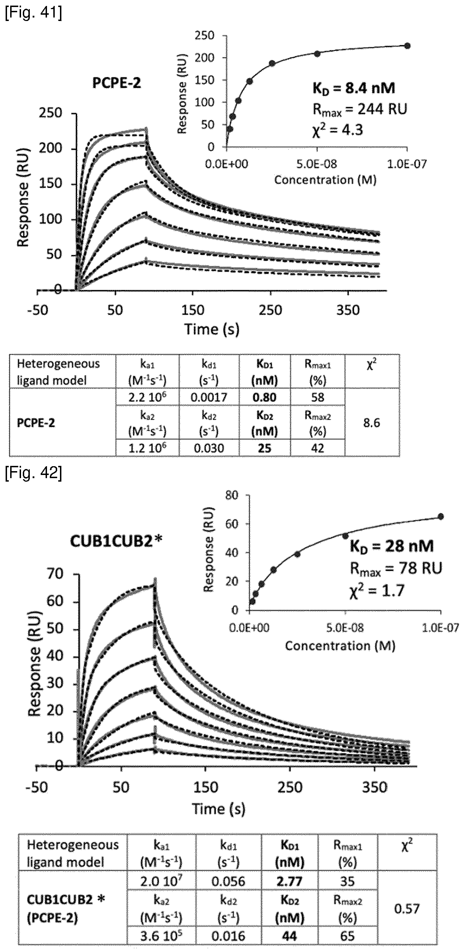

The inventors' next goal was to decipher the mechanism of BMP-1 inhibition by PCPE-2. Using the separate CUB and NTR domains generated above, the inventors first showed that the CUB domains were necessary and sufficient for the inhibition of the cleavage of the fluorogenic peptide and that the NTR domain had no effect (**Figure 36**). On the BMP-1 side, the catalytic domain of the protease could also cleave the peptide but was not affected by the presence of PCPE-2 (**Figure 38**), also excluding the possibility that PCPE-2 acts as a competitive inhibitor binding into the protease active site. In line with these results, PCPE-2 and CUB1CUB2* but not NTR bound to immobilized BMP-1 by SPR (**Figure 39, Figure 41, Figure 42**). Furthermore, full-length BMP-1 but not its catalytic domain bound to immobilized PCPE-2 (**Figure 40**). The dissociation constant for the interaction of PCPE-2 with BMP-1 was found to be 8.4 nM with the steady-state fit (Fig. 4f), in the same range as the Kiapp measured above and as the dissociation constant for the interaction of PCPE-2 with procollagens (**Figure 19**). In the same conditions, PCPE-1 only gave a very weak signal (**Figure 39**), as previously described. Therefore, the inventors could hypothesize that the difference in activity between the two proteins comes from the increased ability of PCPE-2 to form a stable complex with BMP-1, an interaction which further leads to BMP-1 inhibition.

Interactions of PCPE-2 with procollagen and BMP-1 are mutually exclusive.

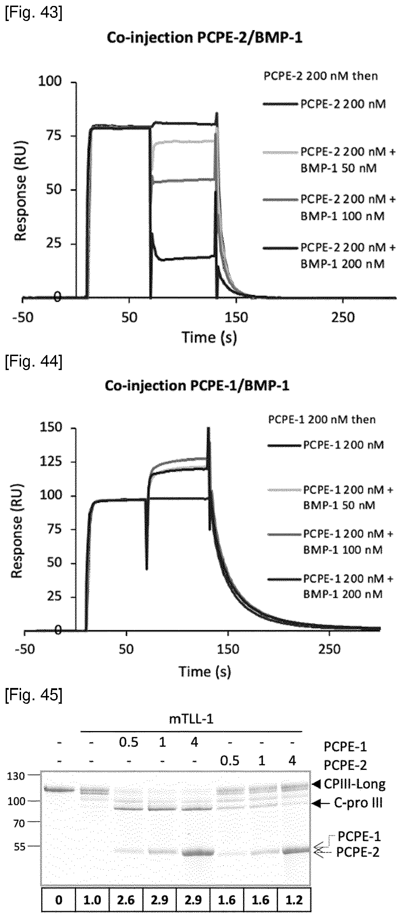

To better understand the consequences of the dual binding capacity of PCPE-2, the inventors ran a competition experiment which consisted of injecting increasing concentrations of BMP-1 on a C-propeptide III surface (an uncleavable substrate mimic) to which PCPE-2 was already bound (**Figure 43**). Interestingly, BMP-1 was able to disrupt the complex between PCPE-2 and C-propeptide III in a concentration-dependent manner, leading to 77 % inhibition when both BMP-1 and PCPE-2 were injected at the same concentration (200 nM). Here again, PCPE-2 behaved differently from PCPE-1 as the simultaneous injection of PCPE-1 and BMP-1 over immobilized C-propeptide III did not lead to a decreased signal but rather to an increased signal (**Figure 44**), revealing that BMP-1 and PCPE-1 could bind together to C-propeptide III. These important results demonstrate that the interactions of PCPE-2 with the procollagen substrate and the BMP-1 protease are mutually exclusive and that a stable ternary complex involving BMP-1, PCPE-2 and a procollagen substrate is unlikely to occur.

PCPE-2 specifically inhibits BTPs but also interferes with the PCP activity of meprins through its interaction with procollagens

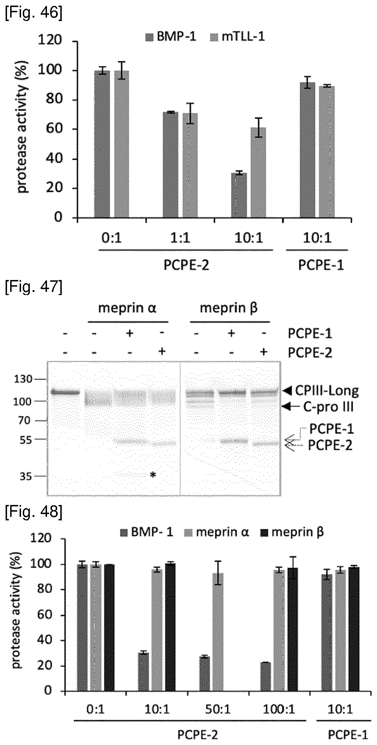

The inventors next wanted to know if PCPE-2 was specific to BMP-1. The inventors first tested its effect on mTLL-1 in a CPIII-Long cleavage assay (**Figure 45**). Interestingly, the enhancement of the PCP activity of mTLL-1 by PCPE-2 followed the same trends as for BMP-1 with a maximum enhancement factor of 1.6 at 0.5:1 and 1:1 molar ratios which then dropped off at higher PCPE-2 concentrations. In addition, PCPE-2 could efficiently inhibit mTLL-1 in the fluorogenic peptide assay (**Figure 46**), albeit to a slightly lower extent than with BMP-1. This indicated that PCPE-2 could affect both the collagenous and non-collagenous substrates of the enzyme, in agreement with what was observed above for BMP-1.

Meprin $\alpha$ and meprin $\beta$ are two other members of the astacin-like subgroup of metalloproteinases that were tested next. They were differentially impacted in the fluorogenic peptide and CPIII-Long assays with no effect of PCPE-2 on the peptide cleavage but a slight inhibition of the maturation of the procollagen-derived protein by meprins (**Figures 47 and 48**). The latter result was in agreement with the inventors' previous finding that PCPE-1 could inhibit the PCP activity of meprins by blocking their access to the cleavage site through its tight interaction with procollagens. A similar mechanism was probably also at play with PCPE-2 but to further confirm that PCPE-2 does not inhibit other meprin activities, another physiological substrate of these proteases (CD99) was tested. As expected, CD99 cleavage was not affected by PCPE-2 (**Figure 49**) and this strongly suggested that PCPE-2 inhibition specifically targets BTPs. Consequently, PCPE-2 appears as the first specific endogenous inhibitor of BTPs in mammals.

DISCUSSION

Most protease families have their own specific inhibitors which protect cellular and extracellular proteins from the deleterious effects of a prolonged or excessive proteolytic activity. The well-described inhibitors of matrix metalloproteinases are the TIMPs (tissue inhibitors of metalloproteinases) but they are not active on BTPs. Meprins were described to be regulated by fetuin-B but again this protein was not effective against BTPs. Here, by demonstrating that PCPE-2 is a potent and specific inhibitor of BTPs targeting all their proteolytic activities, the inventors put an end to the exceptional status of BTPs which seemed to rely on enhancing proteins more than on inhibitors to control their activity. This new situation is actually more coherent with the fact that BTPs are activated during their transit in the Trans-Golgi network and that they are supposedly fully active when they are secreted in the extracellular environment, with no other means than inhibition or degradation to abolish their activity.

The inventors have shown in this study that the broad inhibition of BMP-1 activities by PCPE-2 is mediated by a direct and strong interaction between the regulatory protein and the protease, a mechanism that is not shared by PCPE-1. Rather surprisingly, PCPE-2 has a netrin-like (NTR) domain in common with the TIMPs but its inhibitory activity is not borne by this domain which is fully dispensable for inhibition. In contrast, the CUB domains of PCPE-2 could nicely recapitulate BMP-1 binding and inhibition, suggesting that these domains alone are sufficient. On the protease side, the catalytic domain of BMP-1 alone cannot interact with PCPE-2 and this result indicates that the latter regulates protease activity through an interaction involving its non-catalytic domains. These are also made of CUB domains, organized in a CUB1-CUB2-EGF-CUB3 sequence, and they have been suggested to adopt a closed horseshoe conformation in solution, bringing CUB3 in close proximity to the catalytic domain. However, the precise molecular mechanism explaining why PCPE-2, in contrast to PCPE-1, can form tight complexes with BTPs remains unclear and will require further investigation.

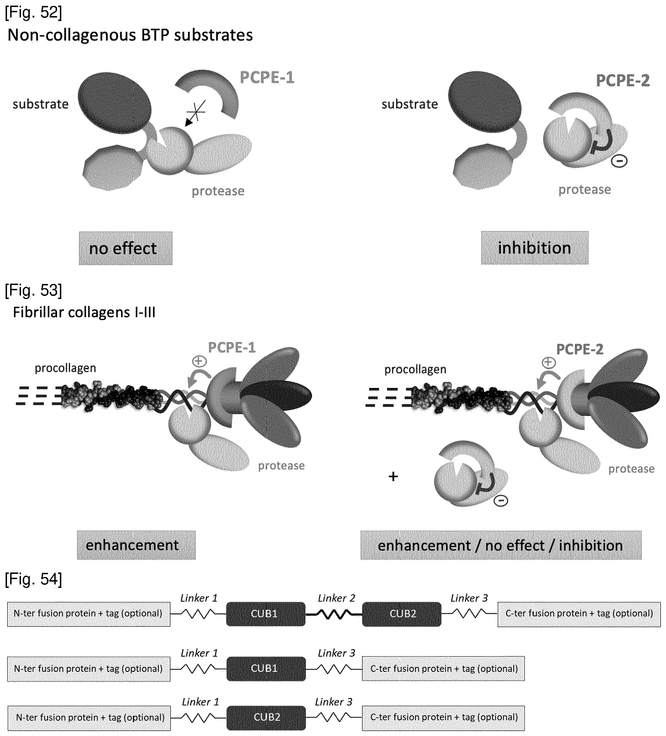

A summary of the finely-tuned regulation of BTP activity permitted by the two PCPEs is presented in **Figures 52 and 53.** The entire range of possible outcomes, from no effect to enhancement and inhibition, is potentially available but the final effect can vary with the nature of the substrate. In the case of non-collagenous substrates (**Figure 52**), the situation is rather straightforward with PCPE-1 having no effect and PCPE-2 behaving as a potent inhibitor of their BTP-dependent cleavage. As a result, only inhibition of proteolytic activity (by PCPE-2) can be observed. In contrast, for fibrillar procollagens, the inventors' results suggest a much more complex situation (**Figure 53**). If previous findings regarding the efficient enhancement of BTP activity by PCPE-1 were fully confirmed in this study, it is not the case for PCPE-2. Even if enhancement of procollagen maturation can be observed in vitro in a specific range of PCPE-2 concentrations, the level of stimulation achieved by this protein is generally much lower than by PCPE-1. This is especially true for procollagens I and III but less for procollagen II, for which the enhancement is more easily observed. Therefore, the use of the procollagen II substrate for the early characterization of PCPE-2 may have led to the partially flawed conclusion that, at least in vitro, PCPE-2 works exactly like PCPE-1. Indeed, the inventors have observed that, in some situations, PCPE-2 (but not PCPE-1) can also inhibit the PCP activity of BMP-1 or have no effect. This unexpected result can be explained by the fact PCPE-2 binds to BMP-1 through the same domains (CUB1CUB2) as those involved in the interaction with collagen C-propeptides, through overlapping binding sites, as demonstrated by competition experiments. This results in mutually exclusive interactions of PCPE-2 with the procollagen or the protease and leads to the unusual bell-shaped curve described above, with successive phases of stimulation by PCPE-2 (through procollagen binding), status quo (equilibrium between stimulation and inhibition) and inhibition by PCPE-2 (through protease binding). At the highest concentrations, PCPE-2 works by hijacking BMP-1 to prevent its interaction with procollagen substrates, independently of the presence of PCPE-1 which then becomes totally powerless. It even appears that PCPE-1 potentiates the inhibition of BMP-1 PCP activity by PCPE-2, probably through the saturation of binding sites on procollagens which makes more PCPE-2 molecules available to bind BMP-1. This is reflected by the higher inhibition of procollagen processing observed when both PCPEs are present than when PCPE-2 alone is present.

An important question related to these findings is whether this complex mechanism of regulation of procollagen processing is relevant in vivo. Based on the relatively low expression of PCOLCE2 in fibroblasts and the absence of major alterations in collagen fibrils, the answer seems to be that PCPE-2 is actually not crucial for proper collagen assembly in homeostatic conditions. However, it cannot be excluded that PCOLCE2 expression might be induced upon challenge. There is presently no evidence for that, at least in conditions known to trigger high collagen synthesis such as tissue injury, and PCOLCE2 does not seem to follow the up-regulation trend often displayed by BMP1 and PCOLCE. Its protein or RNA level was actually found to be down-regulated in murine models of corneal scarring and lung injury and unchanged in a model of cardiac fibrosis. Interestingly also, in skin and kidney, PCOLCE2 expression seems restricted to specific fibroblast subpopulations with progenitor potential rather than with myofibroblast/matrix-producing potential. In contrast, PCOLCE2 is more highly expressed than PCOLCE in human macrophages and could regulate neutrophil functions. Even though the regulation of PCOLCE2 expression during injury-driven inflammation remains to be established, these data suggest that PCPE-1 and PCPE-2 might play distinct and nonoverlapping roles in tissue repair. In line with this, the inventors have observed here that PCPE-2 can efficiently counteract PCPE-1 activity, potentially leading to conflicting outcomes if both are present simultaneously in the same locations. More intriguingly, the inventors' present results do not fit with the findings, described in an earlier report, suggesting that Pcolce2 deficiency protects mice against collagen accumulation and myocardial stiffening in a model of chronic pressure overload. These findings will have to be confirmed and investigated again to determine if they really depend on the direct regulation of BTP-dependent collagen maturation by PCPE-2 or on unrelated mechanisms.

If procollagen processing is not a major target of PCPE-2, other novel potential functions can nonetheless be proposed for this protein. For example, the proteolytic products resulting from some of the substrates characterized in this study are known to play a role in the control of cell adhesion and migration (TSP-1), of TGF-β activation and bioavailability (TSP-1, betaglycan) and/or of angiogenesis (TSP-1, perlecan/endorepellin). Their regulation by PCPE-2 could thus give new mechanistic hints to explain that the latter recently emerged as a signature gene predicting survival in several types of cancers. Similarly, the cleavage of LDLR is strongly inhibited by PCPE-2 suggesting a connection with PCPE-2 relatively well-established roles in the regulation of diet-induced atherosclerosis and adipose tissue expansion. However, these roles were mainly described in mouse models where the LDLR cleavage site that is targeted by BMP-1 in the human protein is not conserved. Other mechanisms are therefore also certainly involved such as a direct interaction of PCPE-2 with other partners (e.g. the scavenger receptor SR-BI, as previously proposed) or the cleavage of other BMP-1 substrates linked to lipid metabolism. In this context, a BMP-1 substrate that was unfortunately not available to us but will require further investigation is pro-ApoA1. Its proteolytic maturation by BMP-1 was previously reported to be enhanced in the presence of PCPE-2 while the inventors' present results would rather predict that it should be inhibited.

In summary, the inventors have shown that with the same combination of two CUB domains sharing more than 50 % identity, it is possible to obtain, on one side, a powerful enhancer of one specific activity of a protease family (PCPE-1) and, on the other side, a potent inhibitor of all the other activities of the same protease family (PCPE-2). Future work should now focus on the identification of the molecular determinants which allow this rather amazing specialization. Also, it will be essential to characterize the cell types that are the most relevant in terms of PCPE-2 production and its possible co-expression with BTPs in time and space in order to better define the scope of the new regulatory mechanisms unveiled by the present study.

MATERIALS AND METHODS

Pcolce2-null mice

Pcolce2-null embryos were obtained from the group of Dr. Thomas Boehm at the Max-Planck-Institute of Immunobiology and Epigenetics in Freiburg, Germany. They were generated by replacing a large part of the exon 3 of the Pcolce2 gene by the IRES-lacZ/neomycin resistance cassette, as previously described. Embryos were injected into C57BL/6 females and the offspring were mated with C57BL/6 mice to generate both wild-type (WT) and Pcolce2-null (KO) mice. These were housed in the pathogen-free facilities of the University of Freiburg or of the SFR Biosciences (Lyon, France) according to ethical regulations.

Transmission electron microscopy

Six week-old KO and WT mice were sacrificed and back skin, heart and tail tendon were carefully dissected and processed for transmission electron microscopy (TEM). Small tissue pieces (around 1 mm3 in size) were fixed in one volume of 4 % glutaraldehyde and one volume of sodium cacodylate 0.2 M pH 7.4 at 4 °C. Then, samples were carefully rinsed 3 times at 4 °C and post-fixed with 2 % OsO4 for 1 h at 4 °C before being dehydrated in graded series of ethanol and transferred to propylene oxide. Impregnation was performed with Epon epoxy resin (Electron Microscopy Sciences). Inclusion was obtained by polymerization at 60 °C for 72 h. Ultrathin sections (approximately 70 nm thick) were cut on a UC7 ultramicrotome (Leica), mounted on 200 mesh copper grids coated with poly-L-lysine (Electron Microscopy Sciences), stabilized for 1 day at room temperature and contrasted with uranyl acetate. Sections were examined with a Jeol 1400JEM 120kV transmission electron microscope (Tokyo, Japan), equipped with a Gatan Orius 600 camera on wide field position and Digital Micrograph software (Gatan Inc).

Fibril diameters were analyzed with imaged v1.53 on 2-6 TEM images/mouse for skin and tendon and up to 10 images/mouse for heart (4-8 mice/genotype). Diameters were derived from Feret diameters for skin fibrils, cross-sectional areas for heart fibrils and diameters of the smallest inscribed circles for tendons.

Skin mechanical properties

Skin mechanical properties were measured using uniaxial traction assays as previously described40. Briefly, skin from the back of 8 week-old WT and KO mice was depilated, the epidermis was removed using 3.8 % ammonium thiocyanate and the remaining dermis was cut into a dog-bone shape along the antero-posterior direction to ensure homogeneous uniaxial tensile load in the central tested portion. The sampled papillary dermis was labelled with a pattern of dots of Indian ink using a soft brush before being attached to the traction device using metallic jaws. The marked surfaces were lit with white light using a LED light (Schott, KL 2500 LED), and images were recorded every 3 s during the test using a digital camera (Allied Vision GX6600) equipped with a telecentric lens (Opto Engineering TC16M036), allowing the observation of the full skin sample. The pattern of Indian ink dots created the macroscopic pattern needed to analyze the experiments with Digital Image Correlation in post treatment using CorrelManuV software.

Prior to the experiment, the dimensions of each sample were measured using a caliper. Tensile tests were performed at a strain rate around 0.5 %s-1, without any stops, until rupture of the sample. During each test, the displacement of the grips and the force were recorded every second. The force was divided by the initial section of the sample to obtain the nominal stress. Stretch was determined either through the machine or through the optical measurement, averaged on the whole sample. Finally, four parameters were extracted from the nominal stress vs stretch curve: the tangent modulus of the linear region, the heel region length, the failure stretch and the ultimate tensile stress.

Protein production

The human PCPE-2 sequence was obtained from the pOTB7-PCPE-2 plasmid purchased from Source BioScience (clone ID 3951739). This sequence contained two mutations (829G>T and 874C>A) which were corrected. The PCPE-2 sequence was then cloned into a pJET1.2/blunt vector using the CloneJET PCR Cloning strategy (Thermo-Fisher). PCPE-2 cDNA was further amplified by PCR with an N-terminal 6His-tag and inserted into the mammalian expression vector pHLsec, through the Agel/Xhol sites, using the In-Fusion cloning strategy (Ozyme). The PCPE-2 3C construct was designed to insert one HRV 3C-protease cleavage site between the 6His-tag and the beginning of PCPE-2 (giving the following N-terminal sequence after signal peptide removal: ETGHHHHHHLEVLFQGPAS - SEQ ID NO: 64) and one HRV 3C-protease cleavage site between amino-acids 270 and 271 of full-length human PCPE-2 (as described in **Figure 24**). The corresponding synthetic gene was ordered from Twist Bioscience after codon optimization. The cDNA was digested with AgeI/XhoI and subcloned into linearized pHLsec. All products were checked by Sanger sequencing (Eurofins).

For the production of PCPE-2 and PCPE-2 3C, (HEK) 293-T cells were seeded in HYPERflask or CellSTACK cell culture vessels (Corning) in DMEM AQ medium (Merck) containing 10 % of fetal bovine serum (FBS, Gibco or Eurobio) and 1 % antibiotic-antimycotic solution (AAS, Merck). At 80-90 % confluency, cells were transfected with pHLsec-6His-PCPE-2 or pHLsec-6His-PCPE-2 3C plasmid, using polyethylenimine (PEI, Merck) as transfection reagent, in DMEM medium with only 2 % of FBS and 1 % of Non-Essential Amino Acids (Merck). Culture medium was collected 3 and 5 days after transfection, centrifuged at 1000 g and mixed with protease inhibitors (0.25 mM Pefabloc (Roth) and 2 mM N-ethylmaleimide (Merck)). The first purification step was on Heparin Sepharose 6 Fast Flow resin (Cytiva) which was prepacked into a column and equilibrated in 20 mM HEPES pH 7.4, 0.15 M NaCl. A linear gradient of NaCl was used to elute the protein (from 0.15 to 2 M NaCl) and the PCPE-2 protein was obtained between 0.5 to 0.8 M of NaCl. Imidazole was then added to the pool of PCPE-2-containing fractions to a final concentration of 5 mM before loading the protein solution on Ni-NTA resin (Qiagen). The resin was washed successively with 20 mM HEPES pH 7.4, 0.6 M NaCl containing 20 mM then 50 mM imidazole and the protein was eluted with a gradient of imidazole from 50 to 500 mM. Fractions containing PCPE-2 were pooled, diluted with 20 mM HEPES pH 7.4 to reduce salt concentration down to 0.25 M and loaded on a HiTRAP Heparin-HP column (Cytiva). The protein was eluted with 0.6 M NaCl in 20 mM HEPES pH 7.4, flash-frozen in the same buffer supplemented with 2.5 mM CaCl2 and 0.1 % of n-octyl-β-D-glucopyranoside (βOG, Roth) and stored at -80 °C until use. The average yield for PCPE-2 production was 0.5 mg per liter of culture.

The CUB1CUB2* and NTR domains of PCPE-2 were generated by cleavage of the PCPE-2 3C protein purified as above until elution from the Ni-NTA column. Then, HRV 3C-protease was added to PCPE-2 3C in a 1/30 w/w ratio and the mixture was incubated for 1 h 30 at room temperature, followed by dialysis overnight at 4 °C in a 7 kDa Slide-a-Lyzer (ThermoFisher) to eliminate imidazole. Removal of uncleaved protein and HRV 3C-protease was achieved by purification on Ni-NTA agarose (0.5 ml) equilibrated in 20 mM HEPES pH 7.4, 0.3 M NaCl, 0.1 % βOG. PCPE-2 fragments were collected in the flow-through and loaded on a HiTRAP Heparin-HP column equilibrated in the same buffer. The CUB1 CUB2* domains were recovered in the flow-through while the NTR domain was eluted with 0.6 M NaCl. Before concentration on Amicon 3 kDa devices (Merck), the NaCl concentration of the CUB1CUB2* solution was also adjusted to 0.6 M and calcium (2.5 mM) was added. Proteins were then flash-frozen and stored at -80 °C until use.

Protein concentrations were determined from the optical density at 280 nm with a Nanodrop 2000 (Thermo Scientific), using absorption coefficients computed with the Expasy ProtParam tool, or from the Bradford assay (Coomassie Plus Assay reagent, Pierce).

Cleavage assays

All cleavage assays with protein substrates were performed at 37 °C in 50 mM HEPES pH 7.4, 5 mM CaCl2 and 0.02 % βOG. The NaCl concentration could vary between assays in the range 0.15 M - 0.25 M but was kept consistent between comparable conditions. All other concentrations and incubation times were as indicated in figure legends. Analysis was by SDS-PAGE with 4-20 % polyacrylamide Criterion gels (BioRad), unless otherwise stated, and Coomassie Blue, Instant Blue (Euromedex) or Sypro Ruby (Merck) staining. For Sypro Ruby staining, a maximum amount of 250 ng of substrate/lane was used to stay in the linear range and gels were analyzed with a Typhoon FLA9500 scanner (blue laser, LPG emission filter; Cytiva).

When applicable, quantification of protein band intensities was made with the ImageQuant TL software v8.2 (Cytiva). Enhancement factors for mini-procollagens and CPIII-Long were calculated as the following ratio: [intensity of Intermediate 1 (1 chain cleaved) + 2 x intensity of Intermediate 2 (2 chains cleaved) + 3 x intensity of C-propeptide] / [3 x (sum of intensities of all cleavage products + procollagen)] and normalized to the BMP-1 alone condition.

Cleavage of the fluorogenic peptide Mca-YVADAPK(Dnp)-OH (20 μM, Covalab) was monitored in the same buffer conditions, as described[26]. Fluorescence was recorded for 20 min (excitation: 320 nm; emission: 405 nm) in a Tecan Infinite M1000 fluorimeter and the slope of the curve was used to derive BMP-1 activity. The apparent inhibition constant (Kiapp) value of PCPE-2 was derived from the data of **Figure 37** using the Morrison equation for tight-binding inhibitors[69] in GraphPad Prism:

[Math 1]

$$\frac{vi}{vo} = 1 - \text{k} * \frac{([E] + [I] + Kiapp) - \sqrt{([E] + [I] + Kiapp)^2 - 4[E][I]}}{2[E]}$$

where vi is the measured velocity in the presence of inhibitor, v0 the velocity in the absence of inhibitor, [E] the total enzyme concentration, [I] the added inhibitor concentration, Kiapp the apparent inhibition constant, and k a constant reflecting partial inhibition.

SPR experiments

Surface plasmon resonance experiments were run with a Biacore T200 apparatus (Cytiva) equipped with the Biacore T200 control software (v3.2.1). Protein ligands were covalently immobilized on Series S sensor chips CM5 (Cytiva) by amine coupling chemistry using the reagents included in the Amine coupling kit (Cytiva). Ligands were diluted in 10 mM sodium acetate pH 5 for mini-procollagen III, C-propeptide III and BMP-1 or in 10 mM HEPES pH 7.4 for PCPE-2. SPR signals were recorded simultaneously on a control channel where the same immobilization procedure was applied, except for the presence of protein ligands. Soluble analytes were injected at 30 or 50 $\mu$l/min at 25 °C after dilution in running buffer (10 mM HEPES pH 7.4, 0.15 M NaCl, 5 mM CaCl2 and 0.05 % P20) for 90 or 120 s, either in high performance or dual inject modes. In co-injection experiments, the first binding partner was injected for 60 s followed by the injection of the two co-injected partners, as indicated, for another 60 s. In all cases, regeneration was achieved with 2 M guanidinium chloride. The proteins used for these experiments were the same as for activity assays except for PCPE-1 which was used as the native form with no His tag in SPR analyses. Finally, best fits of the data in kinetic and steady-state modes were obtained with the Biacore T200 evaluation software (v3.2.1).

Cell culture

293-EBNA cells stably transfected with the empty pCEP4 vector or the pCEP4-sizzled vector were available from previous studies. To generate the pCEP4-PCPE-2 construct, a C-terminal 8His-tag was fused to PCPE-2 in the pJET-PCPE-2 vector by Q5 site-directed mutagenesis (New England Biolabs). After digestion with Acc65i and BamHI, the PCPE-2-8His sequence was introduced into the pCEP4 vector. Cells were transfected using Nanofectin (PAA) as transfection reagent and selected with 300 $\mu$g/ml hygromycin (Merck). All 293-EBNA cell lines were grown in DMEM (Merck) with 10 % FBS and 300 $\mu$g/ml hygromycin. At confluency, cells were kept for 20 h in serum-free medium with no antibiotic and the supernatant was collected, supplemented with protease inhibitors (0.25 mM Pefabloc, 2 mM NEM, 2 mM EDTA), centrifuged and stored at -80 °C. The cell lysate was prepared in RIPA buffer (50 mM Tris pH 8, 0.15 M NaCl, 1 % NP-40, 1 % SDS, 0.5 % sodium deoxycholate) containing protease inhibitors (Complete, Roche) and then centrifuged and stored at -80 °C until use. Protein concentrations in cell extracts were measured with the Bradford assay using the Coomassie Plus Assay reagent (Pierce) and BSA as a standard.

Murine fibroblasts (NMF) were isolated from the back skin of C57BU6 mouse pups by first digesting skin with 1 mg/ml dispase at 4 °C overnight and then mechanically stripping the epidermis from the dermis using forceps. The dermis was collected and processed for fibroblast isolation by mincing it with scissors and incubating it with 500 U/ml collagenase I at 37 °C for 1 h. The digested slurry was passed through a 70 $\mu$m cell strainer, cell pelleted with centrifugation, dissolved in DMEM/F12 (ThermoFisher Scientific), 10% FBS (Merck), 2 mM L-glutamine (Merck) and 1 % AAS and seeded in tissue culture flasks. The cells were maintained in complete DMEM/F12 and cells in passage 1 or 2 used for the analyses. Primary human fibroblasts were isolated by outgrowths of explant cultures from breast and abdominal skin of healthy female donors aged 25-35 years by the Cell and Tissue Bank of Edouard Herriot Hospital (Lyon, France) or the Department of Dermatology, Medical Center of the University of Freiburg (Freiburg, Germany), in accordance with ethical regulations. Human fibroblasts were cultured in complete DMEM/F12 or DMEM AQ™ medium with 10 % FBS and 1 % AAS. Mouse and human fibroblasts were passaged and detached using trypsin-EDTA (PAN Biotech or Merck).

Immunoblotting

Proteins were separated by SDS-PAGE in 25 mM Tris pH 8.5, 0.2 M Glycine, 0.1 % SDS buffer (Euromedex). Proteins were then electroblotted for 2 h onto polyvinylidene difluoride (PVDF; 0.45 $\mu$m) membranes (Millipore) in 10 mM CAPS (N-cyclohexyl-3-aminopropanesulfonic acid) pH 11 with 10 % ethanol. If quantification of total protein amount was required, Stain-Free activation was performed on the acrylamide gel for 30 s using a Fusion FX system (Vilber Lourmat) prior to electroblotting and transferred proteins were visualized using Stain-Free detection on the PVDF membrane after electroblotting. Membranes were then blocked with 10 % skim milk in PBS for 1 h and incubated overnight with primary antibodies diluted into blocking buffer solution (5 % skim milk in PBS with 0.05 % Tween 20) at 4 °C (see details of primary antibodies below). After three washes in PBS containing 0.05 % Tween 20, horseradish peroxidase (HRP)-coupled secondary antibodies (Cell Signaling Technology or Dako; dilutions 1:4000-1:10,000) were added for 1 h at room temperature. Proteins of interest were detected by chemiluminescence with Amersham ECL Select Western blotting detection reagent (Cytiva) using the Fusion FX camera system (Vilber Lourmat). When applicable, quantification of band intensities was performed with Fusion FX software or ImageQuant TL software v8.2 (Cytiva).

Human and mouse C-propeptides of procollagen I were detected with the LF41 antibody70 which was a kind gift of Dr. Larry W. Fisher (NIH, Bethesda, USA). LDLR was detected with AF2148, human PCPE-1 with AF2627, mouse PCPE-1 with AF2239 and His-tagged proteins with MAB050, all from BioTechne. A new antibody was generated in rabbits against a synthetic peptide corresponding to amino-acids 201-230 of human PCPE-2 (DVERDNYCRYDYVAVFNGGEVNDARRIGKY - SEQ ID NO: 62) by the Covalab company (France). Rabbit serum was first purified against the immunogen and then against human PCPE-1 to remove all potential antibodies which could cross-react with PCPE-1. This was stored in PBS with 50 % glycerol at -20 °C.

RNA isolation and quantitative real-time PCR

Total RNA from primary cells was isolated using the RNeasy Plus Mini kit (Qiagen) or the Nucleospin RNA kit (Macherey-Nagel) according to the manufacturer's instructions. RNA from mouse skin was extracted using the RNeasy Fibrous Tissue Mini kit (Qiagen). Quality and quantity of the isolated RNA were determined with a Nanodrop 2000. RNA (500 ng) was then reverse-transcribed to cDNA using the First Strand cDNA Synthesis Kit (ThermoFisher Scientific) or the PrimeScript RT-PCR kit (Takara). Quantitative real-time PCR (qRT-PCR) analyses were performed with a CFX96 Real-Time system (BioRad) or the Rotor-Gene Q system (Qiagen). qRT-PCR reactions were made using the iQ SYBR Green Supermix (BioRad) or the FastStart Universal SYBR Master mix (Roche Applied Science) according to the manufacturers' recommendations with the primers described in Supplementary Table 1 (amplification efficiency > 90 %). PCR conditions were as follows: initial denaturation at 95 °C for 10 min then 50 amplification cycles with denaturation at 95 °C for 10 s and annealing at 60 °C for 20 s. Fluorescence was measured at the end of the annealing step of each cycle and quantification cycles (Cq) were determined for each gene and condition. At the end of the amplification, a melting curve was recorded by heating at 0.5 °C/s from 70 °C to 94 °C. Relative quantification was performed from three technical replicates using the 2-ΔΔCq method, which allows the comparison of two conditions after normalization with the reference genes (GAPDH and Gapdh for human and mouse cells respectively).

PCPE-2 structure modeling and sequence alignments

Sequence alignments and rendering were performed using Clustal Omega (via the Uniprot web server) and ESPript 3.0 (https://espript.ibcp.fr) respectively, based on Uniprot sequences Q15113 and Q9UKZ9 and structural information extracted from PDB entry 6FZV34. Protein structures were drawn using UCSF Chimera (https://www.cgl.ucsf.edu/chimera). YASARA (http://www.yasara.org) was used for homology modeling of PCPE-2 and computing of rmsd.

Statistical analysis

Statistical analyses were performed with GraphPad Prism 8. Using a 5 % threshold, the inventors first verified the normality of the distribution using the Shapiro-Wilk test as well as the equality of variance with the Fisher test. If these tests were passed successfully, the significance of the mean difference was assessed using an unpaired t-test. In other situations, the test used was as indicated in figure legends. For multiple comparisons, one-way ANOVA with paired data and Dunnett post-test was used.

Example 2: Mutants of the peptides according to the invention.

The inventors also produced polypeptides derived from PCPE-2 CUB1 and CUB2 domains as inhibitors of BMP-1/tolloid-like proteases (BTPs) to treat pathologies in which these proteases are involved. BTPs play a major role in the assembly of the extracellular matrix (in particular in collagen fibrillogenesis), the regulation of TGF-beta activity, angiogenesis, lipid metabolism, muscle growth and the development of the peripheral nervous system.

The pathologies that could benefit from their effective inhibition therefore include fibrosis, cancer, metabolic diseases (atherosclerosis, diabetes, NASH) and muscular disorders (as described in myopathies, cachexia or aging).

The Inventors have demonstrated that the human PCPE-2 protein is a potent (apparent Ki < 3 nM) and selective inhibitor of BTPs (no inhibition of the related meprin α and β proteases). The domains involved in inhibition are the CUB1 and CUB2 domains of PCPE-2. Human PCPE-2 protein, its CUB1CUB2 domains (native or mutated at certain positions) or peptides derived from the CUB1 and CUB2 domains are thus produced as inhibitors of BTP proteases, alone or in combination with other drugs, to prevent or treat the diseases listed above.

Products developed

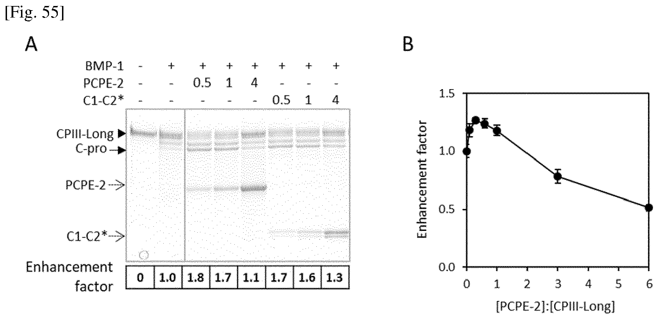

The inventors characterised a protein called PCPE-2 (procollagen C-proteinase enhancer 2) which is very similar to PCPE-1, the activator of collagen maturation mentioned above (43% sequence identity), and which until now had been described as having the same activity. In collagens I and III, which are relevant in the context of fibrosis and most cancers, there is indeed a slight activation in the presence of low concentrations of PCPE-2, but this evolves towards inhibition at higher concentrations (**Figure 55**).

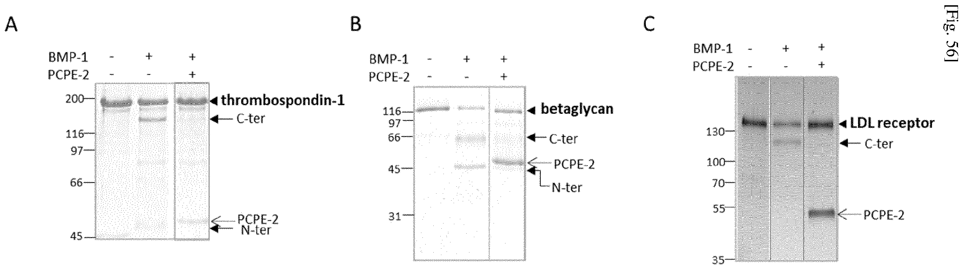

This dual or biphasic behaviour is in fact due to the most interesting property of PCPE-2, which is its ability to interact strongly with BTP proteases to induce their inhibition, a property not shared with PCPE-1. This translates spectacularly on non-collagenous substrates into highly effective inhibition of their cleavage by BTPs (**Figure 56**).

The inventors have shown that the active part of PCPE-2 is contained in the CUB1 and CUB2 domains and that a truncated CUB1CUB2 (or C1-C2) protein has the same inhibitory activity. The inventors therefore propose to use these domains (**Figure 54**) to develop a potent and specific inhibitor of BTPs that could be used for a variety of therapeutic applications. Individual CUB1 (C1) or CUB2 (C2NTR) domains are still capable of binding BMP-1 but are only weakly inhibitory. If each of these domains interacts with a specific BMP-1 domain, as is likely for this type of protein, it could also be interesting to associate them with other proteins, such as antibody fragments or protein binders, with particular specificities for BTP domains not targeted by PCPE-2 (**Figure 54**).

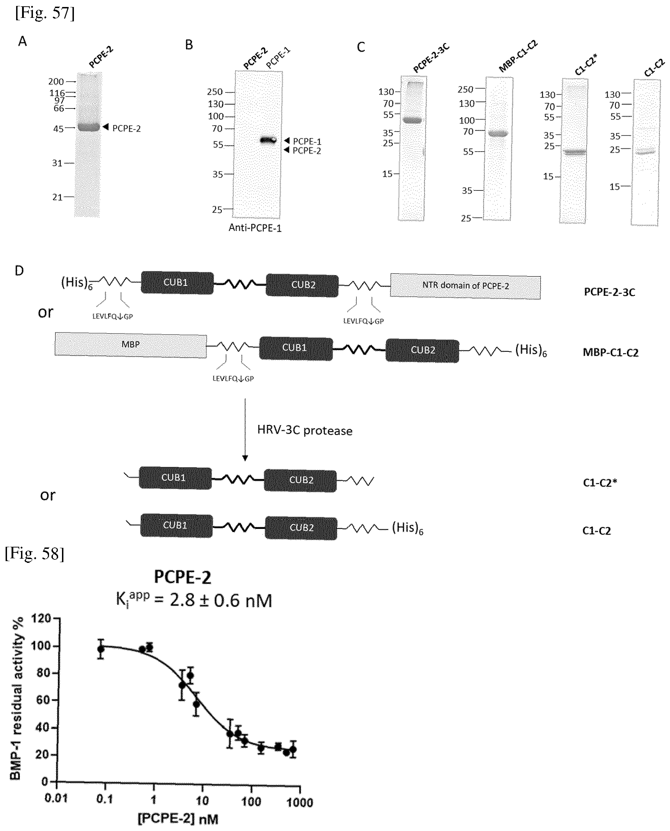

Finally, the whole PCPE-2 protein can be produced in a relatively stable form and in good yield (0.5-5 mg/l) in mammalian cells (HEK 293-T and 293-F cells). It should be stored in an optimised buffer such as 20 mM HEPES pH 7.4, 0.5 M NaCl, 2.5 mM CaCl2, 0.1 % n-octyl-β-D-glucopyranoside, which is non-toxic as it has already been used for injections in llamas. Isolated CUB domains are more difficult to obtain and the inventors have developed a production system in the form of cleavable fusions with various proteins such as MBP (Maltose-Binding Protein), mVenus or even the native NTR domain of PCPE-2 (PCPE-2-3C) (**Figure 57**)**.**

Characterisation

PCPE-2 and the C1-C2 or C1-C2* proteins (or their fusions) can be produced with excellent purity without contamination by PCPE-1 in mammalian cells, in the presence of 2% serum (HEK 293-T) or in defined medium (HEK 293-F) (**Figure 57**)**.**

PCPE-2 inhibits the cleavage of non-collagenous substrates of the BTPs tested here in the presence of the most ubiquitous protease of the family (BMP-1). Some examples are given in **Figure 56,** but all the cleavages tested so far are inhibited. Inhibition of thrombospondin-1 and betaglycan cleavage, for example, is relevant in the context of tumour progression because the products of these cleavages regulate TGF-β signalling and angiogenesis. Inhibition of LDL receptor (LDLR) cleavage is relevant in the context of atherosclerosis and metabolic diseases.

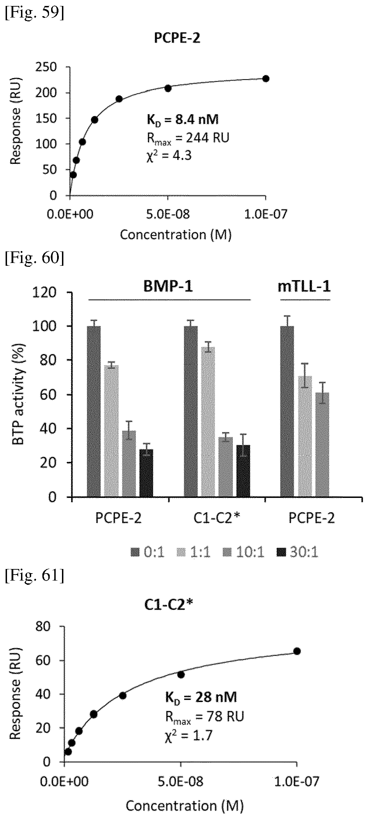

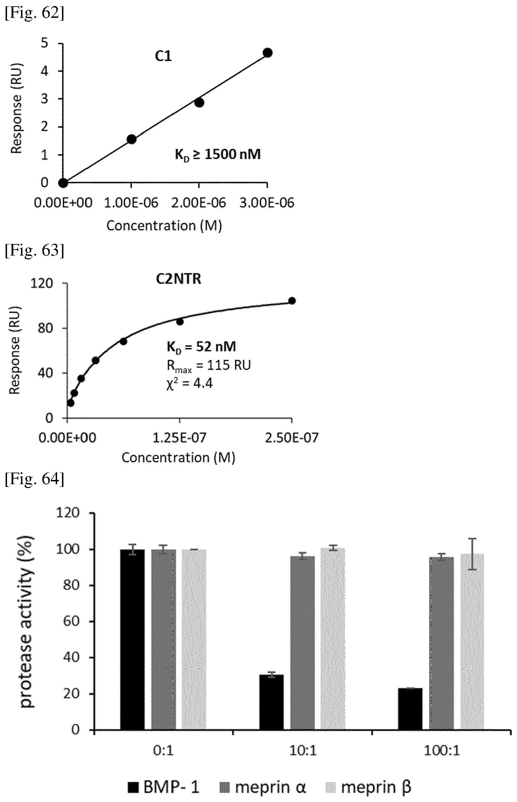

PCPE-2 inhibits the cleavage of a fluorogenic peptide substrate of BTPs with an apparent $K_i$ of 2.8 +/- 0.6 nM in the case of the protease BMP-1 (**Figure 58**). It binds BMP-1 with a dissociation constant of 8.4 nM (**Figure 59**). C1-C2* inhibits fluorogenic peptide cleavage as effectively as PCPE-2 (**Figure 60**) and binds BMP-1 with a KD of 28 nM (**Figure 61**). Individual CUB domains (C1 and C2NTR) also interact with BMP-1 but with lower affinities (**Figures 62 and 63)**. PCPE-2 also inhibits mTLL-1, another member of the BTP family (**Figure 60**)**.** However, it has no effect on the BTP-related proteases meprins (α and β; **Figure 64**) and at this stage appears to be specific to BTPs. BTPs and meprins belong to the same subfamily of metalloproteinases (astacins) within the extracellular metalloproteinases. Although this has not been demonstrated, it is therefore unlikely that PCPE-2 can inhibit other proteases.

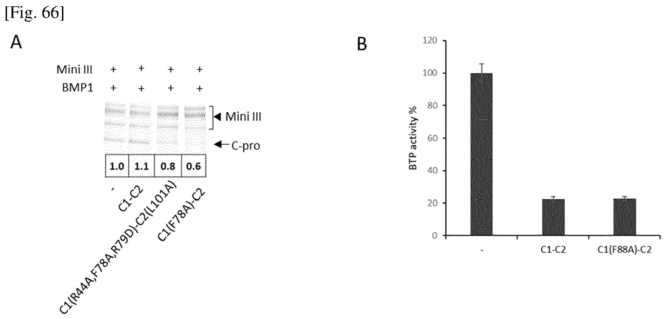

The weak point of PCPE-2 from the point of view of a possible therapeutic application is its dual activity on fibrillar collagens I and III, slightly activating at low concentration and inhibiting at high concentration **(Figure 55)**. This activity cannot be controlled in vivo and could lead to harmful side effects. The inventors therefore modified the C1-C2 proteins at certain positions (R44, F78 and R79 in CUB1 and L101 in CUB2) in an attempt to abolish their ability to interact with procollagens (sequences 12-22, individual or combined mutations) while retaining interaction with BTPs. The F78A and R79D mutations have already produced a significant change in activity (**Figure 65**)**.** Furthermore, the interaction of C1-C2 with procollagens is completely abolished with the combination of the 4 mutations, without the interaction with BMP-1 being significantly affected (**Figure 65**)**.** This translates into a loss of the ability to activate C-terminal maturation of procollagens but not to inhibit BTPs on a fluorogenic substrate **(Figure 66**).

Therapeutic tool

Given its ability to inhibit the action of BTPs, the PCPE-2 protein (or its derived polypeptides) has therapeutic potential in fibrosis, cancer, muscular disorders and metabolic disorders. Local targeting should be favoured insofar as BTPs are essential to numerous physiological processes.

It should also be noted that under-expression of PCPE-2 has been associated with the development of diabetes, fibrosis of the lung and liver and breast cancer (TCGA data, http://ualcan.path.uab.edu/cgi-bin/TCGAExResultNew2.pl?genenam=PCOLCE2&ctype=BRCA), diseases in which a supply of PCPE-2 or derivatives could therefore be of interest.

Small molecule inhibitors of BTPs already exist, mainly developed by manufacturers such as Pfizer and Glaxo (US7214694; WO2017/006296), but their specificity in relation to meprins and other metalloproteases is not always optimal. The Sizzled protein, found in xenopus and zebrafish but not in mammals, has also been described as a highly potent and selective inhibitor of BTPs, but may pose immunogenicity problems if injected into humans. More recently, antibodies capable of blocking the various BTP enzymes have been developed (WO2008/011193A2; WO2013/163479A1; WO2022/024034A1), suggesting that it is relevant to target these proteases in the context of fibrosis and muscle disorders.

However, to date, none of these compounds has resulted in a clinically-used drug. The use of proteins or peptides derived from PCPE2 should enable the development of an effective and selective compound, without immunogenicity problems, and of a reasonable size for administration (the size of C1-C2 is 30 kDa).

Applications

Prevention and treatment of fibrosis of internal organs, particularly fibrosis following an acute event (infarction, infection, intoxication); preventive treatment of corneal opacities following refractive corneal surgery; treatment of localised skin wounds in areas under mechanical stress; cancers (subtypes to be defined); cachexia.

MATERIALS AND METHODS

Protein production

The human PCPE-2 sequence was first cloned into a pJET1.2/blunt vector using the CloneJET PCR Cloning strategy (ThermoFisher). PCPE-2 cDNA was further amplified by PCR with an N-terminal 6His tag and inserted into the mammalian expression vector pHLsec, through the AgeI/XhoI sites, using the In-Fusion cloning strategy (Ozyme). CUB2NTR was amplified from the PCPE-2 construct in pJET and inserted into pHLsec using the same strategy. The PCPE-2-3C construct was designed to insert one HRV 3C-protease cleavage site between the 6His tag and the beginning of PCPE-2 and one HRV 3C-protease cleavage site between amino-acids 270 and 271 of full-length human PCPE-2 (**Figure 57**). The corresponding synthetic gene was ordered from Twist Bioscience after codon optimization. The cDNA was digested with AgeI/XhoI and subcloned into linearized pHLsec. The N-terminal fusion of PCPE-2 CUB domains with maltose-binding protein (MBP-C1-C2, MBP-C1 and derived mutants) was obtained by subcloning synthetic C1-C2 or C1 constructs with N-terminal HRV 3C-protease cleavage site (obtained from Geneart) into the pHLmMBP-1 vector (Addgene) through the NotI and XhoI restriction sites. The C-terminal fusion of PCPE-2 CUB domains with mVenus (C1-C2-mVenus) was obtained through the simultaneous insertion of C1-C2 and mVenus constructs, digested respectively with AgeI/NheI and NheI/KpnI, into the linearized pHLsec vector. All products were checked by Sanger sequencing (Eurofins).

For the production of PCPE-2 and other PCPE-2-derived polypeptides, (HEK) 293-T cells were seeded in HYPERflask or CellSTACK cell culture vessels (Corning) in DMEM AQ medium (Merck) containing 10 % of fetal bovine serum (FBS, Gibco or Eurobio) and 1 % antibiotic-antimycotic solution (AAS, Merck). At 80-90 % confluency, cells were transfected with the plasmids described above, using polyethylenimine (PEI, Merck) as transfection reagent, in DMEM medium with only 2 % of FBS and 1 % of Non-Essential Amino Acids (Merck). Alternatively, the proteins were produced in (HEK) 293-F cells grown in suspension in FreeStyle 293 expression medium (Gibco) using sterile flasks (Corning or BD Biosciences) placed on an orbital shaker platform (Eppendorf) rotating at 125 rpm at 37 °C with 8 %. On the day of transfection, cells were centrifuged and resuspended at a cell density of 1.106 cells/mL in FreeStyle 293 expression medium. For transfection, plasmid DNA and PEI 25K transfection agent were first diluted separately in 1/20 of the total culture volume of Opti-MEM medium (Gibco), filtered on 0.2 $\mu$m filters (Millipore) for the transfection agent and kept at room temperature for 5 min before mixing. The mixture was further incubated for 15 min before addition to the cells. Finally, 1/5 of the culture volume of fresh medium was added to the cells 24 hours after transfection.

Culture medium was collected between 3 and 5 days after transfection, centrifuged at 1000 g and mixed with protease inhibitors (0.25 mM Pefabloc (Roth) and 2 mM N-ethylmaleimide (Merck)). The first purification step for PCPE-2 and PCPE-2-3C was on Heparin Sepharose 6 Fast Flow resin (Cytiva) which was prepacked into a column and equilibrated in 20 mM HEPES pH 7.4, 0.15 M NaCl. A linear gradient of NaCl was used to elute the protein (from 0.15 to 2 M NaCl) and the PCPE-2 protein was obtained between 0.5 to 0.8 M of NaCl. Imidazole was then added to the pool of PCPE-2-containing fractions to a final concentration of 5 mM before loading the protein solution on Ni-NTA resin (Qiagen). The resin was washed successively with 20 mM HEPES pH 7.4, 0.6 M NaCl containing 20 mM then 50 mM imidazole and the protein was eluted with a gradient of imidazole from 50 to 500 mM. Fractions containing PCPE-2 were pooled, diluted with 20 mM HEPES pH 7.4 to reduce salt concentration down to 0.25 M and loaded on a HiTRAP Heparin-HP column (Cytiva). The protein was eluted with 0.6 M NaCl in 20 mM HEPES pH 7.4, flash-frozen in the same buffer supplemented with 2.5 mM CaCl2 and 0.1 % of n-octyl-β-D-glucopyranoside (βOG, Roth) and stored at -80 °C until use.

C1-C2-mVenus, MBP-C1-C2, MBP-C1 and their mutants were purified through their His-tag on Ni Sepharose excel (Cytiva). Cell supernatant was loaded on the resin equilibrated in 20 mM HEPES pH 7.4, 0.5 M NaCl (buffer A). Then, the resin was washed successively with buffer A and buffer A containing 25 mM imidazole and the protein was eluted with a gradient of imidazole from 25 to 500 mM. Fractions containing the protein were pooled and submitted to size exclusion chromatography on a Superdex 200 increase (Cytiva) with 20 mM HEPES pH 7.4, 0.5 M NaCl, 2.5 mM CaCl2 as equilibration buffer.

C1-C2, C1-C2*, C1 and their mutants were generated by cleavage of the corresponding MBP-C1-C2, PCPE-2-3C or MBP-C1 protein, purified as above until elution from the Ni-affinity chromatography. HRV 3C-protease was added in a 1/30 w/w ratio and the mixture was incubated for 1 h 30 at room temperature, followed by dialysis overnight at 4 °C in a 7 kDa Slide-a-Lyzer device (ThermoFisher) or by desalting on Zeba Spin columns (ThermoFisher) to eliminate imidazole. Purification of cleavage mixtures was achieved on Ni-NTA agarose equilibrated in 20 mM HEPES pH 7.4, 0.5 M NaCl, 2.5 mM CaCl2, 5 mM Imidazole. C1-C2 was recovered in the elution fractions and submitted to buffer exchange using a Zeba Spin desalting column while C1-C2* was collected in the flow-through and further loaded on a HiTRAP Heparin-HP column equilibrated in the same buffer. The C1-C2* polypeptide was recovered in the flow-through. Proteins were then flash-frozen and stored at -80 °C until use.

Human BMP-1 (fused to a C-terminal Flag tag), human mTLL-1 (fused to a C-terminal 8His tag), human PCPE-1 (fused to a C-terminal 8His tag), mini-procollagen III (fused to a N-terminal c-myc tag) were produced in human embryonic kidney (HEK) 293-EBNA cells and purified as previously described. CPIII-Long was obtained and purified from 293-T cells. Rat betaglycan was prepared with the baculovirus/High five cells system. Recombinant human meprin α and meprin β were also expressed in this system and were purified and activated as described previously. TSP-1 was purified from human platelets as described. Finally, the ectodomain of human LDLR (ref. 2148-HP) was purchased from BioTechne.

Cleavage assays

All cleavage assays with protein substrates were performed at 37 °C in 50 mM HEPES pH 7.4, 5 mM CaCl2 and 0.02 % βOG. The NaCl concentration could vary between assays in the range 0.15 M - 0.25 M but was kept consistent between comparable conditions. Analysis was by SDS-PAGE and Coomassie Blue, Instant Blue (Euromedex) or Sypro Ruby (Merck) staining. Sypro Ruby-stained gels were analyzed with a Typhoon FLA9500 scanner (blue laser, LPG emission filter; Cytiva).

When applicable, quantification of protein band intensities was made with the ImageQuant TL software v8.2 (Cytiva). Enhancement factors for mini-procollagen III and CPIII-Long were calculated as the following ratios: [intensity of Intermediate 1 (1 chain cleaved) + 2 x intensity of Intermediate 2 (2 chains cleaved) + 3 x intensity of C-propeptide] / [3 x (sum of intensities of all cleavage products + procollagen)] and normalized to the protease alone condition.

Cleavage of the fluorogenic peptide Mca-YVADAPK(Dnp)-OH (20 μM, Covalab) was monitored in the same buffer conditions. Fluorescence was recorded for 20 min (excitation: 320 nm; emission: 405 nm) in a Tecan Infinite M1000 fluorimeter and the slope of the curve was used to derive protease activity. The apparent inhibition constant (Kiapp) value of PCPE-2 was calculated using the Morrison equation for tight-binding inhibitors in GraphPad Prism.

SPR experiments

Surface plasmon resonance experiments were run with a Biacore T200 apparatus (Cytiva) equipped with the Biacore T200 control software (v3.2.1). Protein ligands were covalently immobilized on Series S sensor chips CM5 (Cytiva) by amine coupling chemistry using the reagents included in the Amine coupling kit (Cytiva). Ligands were diluted in 10 mM sodium acetate pH 5. SPR signals were recorded simultaneously on a control channel where the same immobilization procedure was applied, except for the presence of protein ligands. Soluble analytes were injected at 50 μl/min at 25 °C after dilution in running buffer (10 mM HEPES pH 7.4, 0.15 M NaCl, 5 mM CaCl2 and 0.05 % P20) for 90 s. Regeneration was achieved with 2 M guanidinium chloride. Finally, best fits of the data in steady-state modes were obtained with the Biacore T200 evaluation software (v3.2.1).

Immunoblotting

Proteins were separated by SDS-PAGE in 25 mM Tris pH 8.5, 0.2 M Glycine, 0.1 % SDS buffer (Euromedex). Proteins were then electroblotted for 2 h onto polyvinylidene difluoride (PVDF; 0.45 μm) membranes (Millipore) in 10 mM CAPS (N-cyclohexyl-3-aminopropanesulfonic acid) pH 11 with 10 % ethanol. Membranes were blocked with 10 % skim milk in PBS for 1 h and incubated for 1 h with primary antibody (AF2627, BioTechne) diluted into blocking buffer solution (5 % skim milk in PBS with 0.05 % Tween 20). After three washes in PBS containing 0.05 % Tween 20, horseradish peroxidase (HRP)-coupled secondary anti-goat antibody (Dako) was added for 1 h. PCPE-1 was detected by chemiluminescence with Amersham ECL Select Western blotting detection reagent (Cytiva) using the Fusion FX camera system (Vilber Lourmat).

**Claims**

1. A peptide comprising, consisting essentially, or consisting of the amino acid sequence SEQ ID NO :1

   <u>C</u>GGILTGESGFIGSEGFPGVYPPNSK<u>C</u>TWKITVPEGKVVVLNF**R**FIDLESDNL<u>C</u>RYDFVD VYNGHANGQRIGRF<u>C</u>GT**FR**PGALVSSGNKMMVQMISDANTAGNGFMAMFSAA (SEQ ID NO:1)

   provided that said peptide does not consist in SEQ ID NO: 2, or SEQ ID NO: 3
   or any variant thereof having at least 36% identity with SEQ ID NO: 1 or SEQ ID NO: 3, provided

   i) that the underlined cysteines (C) in SEQ ID NO: 1 are conserved, and that the variant does not contain another cysteine, and
   ii) that said variant retains the ability to bind and/or inhibit a BMP-1/tolloid-like protease or BTP.

2. The peptide according to claim 1, wherein at least one of the amino acids in position 15, 16, 19, 20, 23, 24, 44, 45, 47, 48, 50, 51, 52, 53, 55, 56, 76, 77, 78, 79, 98, 99, 100, 101, 102 and 103 of SEQ ID NO: 1 is substituted by any other amino acid.

3. The peptide according to claim 1 or 2, wherein said peptide comprises both amino acid sequences SEQ ID NO: 1 and SEQ ID NO: 4.

4. The peptide according to anyone of claims 1 to 3, wherein the at least one of the amino acids in position 21, 52, 53, 54, 56, 57, 70, 73, 74, 75, 76, 77, 80, 81, 82, 84, 86, 101, 102, 103, 104 and 106 of SEQ ID NO: 4 is substituted by any other amino acid.

5. The peptide according to anyone of claims 1 to 4, wherein SEQ ID NO: 1 and SEQ ID NO: 4 are directly linked by a linker.

6. The peptide according to anyone of claims 1 to 4, wherein, from the N-terminus to the C-terminus of the peptide, the sequence of the peptide comprises SEQ ID NO: 1, the C-terminus part of SEQ ID NO: 1 is directly linked to the N-terminus part of the linker, and the C-terminus part of the linker is directly linked to the N-terminus part of SEQ ID NO: 4.

7. The peptide according to claim 5 or 6, wherein the linker is chosen from the group comprising SEQ ID NO: 5 or SEQ ID NO: 6 or (GS)n wherein n varies from 1 to 20 or (GGGS)m wherein m varies from 1 to 10.

8. The peptide according to anyone of claims 1 to 7, wherein the peptide comprises a sequence able to interact with one of the BTPs.

9. The peptide according to anyone of claims 1 to 8, the peptide comprising, consisting essentially or consisting of one of the following sequences: SEQ ID NO: 7 to SEQ ID NO: 59.

10. A pharmaceutical composition comprising the peptide according to anyone of claims 1 to 9, in association with a pharmaceutically acceptable vehicle.

11. The peptide according to anyone of claims 1 to 9, or the pharmaceutical composition according to claim 10, as medicine.

12. A peptide according to anyone of claims 1 to 9, or the pharmaceutical composition according to claim 10, for its use for the treatment and the prevention of a pathology involving deregulation of a BTP protease.

13. The peptide for its use according to claim 12, wherein the pathology involving deregulation of BTP proteases is chosen from fibrotic disorders, cancers, metabolic diseases including atherosclerosis, diabetes and nonalcoholic steatohepatitis, and myopathies-, cachexia- or aging-associated muscular troubles.

14. Use of a peptide according to anyone of claims 1 to 9, for inhibiting in vitro a BTP.

15. A kit comprising

- a peptide according to anyone of claims 1 to 9, and
- a medicine.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

[Fig. 17]

[Fig. 18]

[Fig. 19]

[Fig. 20]

| Heterogeneous ligand model | $k_{a1}$ $(M^{-1}s^{-1})$ | $k_{d1}$ $(s^{-1})$ | $K_{D1}$ (nM) | $R_{max1}$ (%) | $\chi^2$ |
|---|---|---|---|---|---|
| | $7.9\ 10^6$ | 0.0043 | 0.55 | 61 | |
| PCPE-1 | $k_{a2}$ $(M^{-1}s^{-1})$ | $k_{d2}$ $(s^{-1})$ | $K_{D2}$ (nM) | $R_{max2}$ (%) | 0.43 |
| | $4.8\ 10^6$ | 0.026 | 5.4 | 39 | |

[Fig. 21]

| 1:1 binding model | $k_{a1}$ $(M^{-1}s^{-1})$ | $k_{d1}$ $(s^{-1})$ | $K_{D1}$ (nM) | $R_{max1}$ (RU) | $\chi^2$ |
|---|---|---|---|---|---|
| PCPE-2 | $3.6\ 10^6$ | 0.16 | 45 | 82 | 0.91 |

[Fig. 22]

PCPE-2 200 nM then

— PCPE-2 200 nM

— PCPE-2 400 nM

— PCPE-2 200 nM +
PCPE-1 200 nM

[Fig. 23]

PCPE-1 200 nM then

— PCPE-1 200 nM

— PCPE-1 400 nM

— PCPE-1 200 nM +
PCPE-2 200 nM

[Fig. 24]

FRPK$^{270}$ $^{271}$KLPT - *SEQ ID NO: 63*
LEVLFQ↓GP

3C-protease

CUB1CUB2*

+ NTR

[Fig. 25]

[Fig. 26]

[Figure 27]

**CUB1CUB2***

| 1:1 binding model | $k_{a1}$ $(M^{-1}s^{-1})$ | $k_{d1}$ $(s^{-1})$ | $K_{D1}$ (nM) | $R_{max1}$ (RU) | $\chi^2$ |
|---|---|---|---|---|---|
| CUB1CUB2* (PCPE-2) | $9.7\ 10^6$ | 0.30 | **31** | 52 | 0.02 |

[Fig. 28]

[Fig. 29]

[Fig. 30]

[Fig. 31]

[Fig. 32]

[Fig. 33]

[Fig. 34]

[Fig. 35]

[Fig. 36]

[Fig. 37]

$$K_i^{app} = 2.8 \pm 0.6 \text{ nM}$$

[Fig. 38]

[Fig. 39]

[Fig. 40]

[Fig. 41]

PCPE-2

$K_D$ = 8.4 nM
$R_{max}$ = 244 RU
$\chi^2$ = 4.3

| Heterogeneous ligand model | $k_{a1}$ ($M^{-1}s^{-1}$) | $k_{d1}$ ($s^{-1}$) | $K_{D1}$ (nM) | $R_{max1}$ (%) | $\chi^2$ |
|---|---|---|---|---|---|
| | $2.2 \times 10^6$ | 0.0017 | **0.80** | 58 | |
| PCPE-2 | $k_{a2}$ ($M^{-1}s^{-1}$) | $k_{d2}$ ($s^{-1}$) | $K_{D2}$ (nM) | $R_{max2}$ (%) | 8.6 |
| | $1.2 \times 10^6$ | 0.030 | **25** | 42 | |

[Fig. 42]

CUB1CUB2*

$K_D$ = 28 nM
$R_{max}$ = 78 RU
$\chi^2$ = 1.7

| Heterogeneous ligand model | $k_{a1}$ ($M^{-1}s^{-1}$) | $k_{d1}$ ($s^{-1}$) | $K_{D1}$ (nM) | $R_{max1}$ (%) | $\chi^2$ |
|---|---|---|---|---|---|
| | $2.0 \times 10^7$ | 0.056 | **2.77** | 35 | |
| CUB1CUB2 * (PCPE-2) | $k_{a2}$ ($M^{-1}s^{-1}$) | $k_{d2}$ ($s^{-1}$) | $K_{D2}$ (nM) | $R_{max2}$ (%) | 0.57 |
| | $3.6 \times 10^5$ | 0.016 | **44** | 65 | |

[Fig. 43]

**Co-injection PCPE-2/BMP-1**

[Fig. 44]

**Co-injection PCPE-1/BMP-1**

[Fig. 45]

[Fig. 46]

[Fig. 47]

[Fig. 48]

[Fig. 49]

[Fig. 50]

[Fig. 51]

[Fig. 52]
Non-collagenous BTP substrates

[Fig. 53]
Fibrillar collagens I-III

[Fig. 54]

[Fig. 55]

[Fig. 56]

[Fig. 57]

[Fig. 58]

[Fig. 59]

**PCPE-2**

$K_D$ = 8.4 nM
$R_{max}$ = 244 RU
$\chi^2$ = 4.3

[Fig. 60]

■ 0:1  ■ 1:1  ■ 10:1  ■ 30:1

[Fig. 61]

**C1-C2***

$K_D$ = 28 nM
$R_{max}$ = 78 RU
$\chi^2$ = 1.7

[Fig. 62]

C1

$K_D \geq 1500$ nM

[Fig. 63]

C2NTR

$K_D = 52$ nM
$R_{max} = 115$ RU
$\chi^2 = 4.4$

[Fig. 64]

■ BMP-1　■ meprin α　▨ meprin β

[Fig. 65]

[Fig. 66]

A

B

Europäisches
Patentamt

European
Patent Office

Office européen
des brevets

# EUROPEAN SEARCH REPORT

Application Number

EP 23 30 6041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TALANTIKITE MAYA ET AL: "Inhibitors of BMP-1/tolloid-like proteinases: efficacy, selectivity and cellular toxicity", FEBS OPEN BIO, vol. 8, no. 12, 1 December 2018 (2018-12-01), pages 2011-2021, XP055909461, US ISSN: 2211-5463, DOI: 10.1002/2211-5463.12540 Retrieved from the Internet: URL:https://www.ncbi.nlm.nih.gov/pmc/articles/PMC6275283/pdf/FEB4-8-2011.pdf> * table 1 * | 1-15 | INV. C07K14/81 A61K38/57 A61P19/02 A61P35/00 |
| X | WO 2021/189053 A1 (UNIV YALE [US]) 23 September 2021 (2021-09-23) | 1,3,8,9 | |
| A | * sequence 3040 * | 2,4-7, 10-15 | |
| A | MOTT J D ET AL: "Post-transcriptional proteolytic processing of procollagen C-terminal proteinase enhancer releases a metalloproteinase inhibitor", JOURNAL OF BIOLOGICAL CHEMISTRY, AMERICAN SOCIETY FOR BIOCHEMISTRY AND MOLECULAR BIOLOGY, US, vol. 275, no. 2, 14 January 2000 (2000-01-14), pages 1384-1390, XP002972173, ISSN: 0021-9258, DOI: 10.1074/JBC.275.2.1384 * figure 4 * | 1-15 | TECHNICAL FIELDS SEARCHED (IPC) A61K A61P C07K |

-/--

The present search report has been drawn up for all claims

| Place of search | Date of completion of the search | Examiner |
|---|---|---|
| The Hague | 6 November 2023 | Schmitz, Till |

EPO FORM 1503 03.82 (P04C01)

Europäisches
Patentamt

European
Patent Office

Office européen
des brevets

## EUROPEAN SEARCH REPORT

**Application Number**

**EP 23 30 6041**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE UniProt [Online]<br><br>13 November 2019 (2019-11-13),<br>"SubName: Full=Procollagen C-endopeptidase enhancer 2<br>{ECO:0000313\|EMBL:KAF7484599.1};",<br>XP002810430,<br>retrieved from EBI accession no.<br>UNIPROT:A0A5E4C7T9<br>Database accession no. A0A5E4C7T9<br>* sequence * | 1-15 | |
| A | WO 00/32221 A2 (GENENTECH INC [US]; ASHKENAZI AVI J [US] ET AL.)<br>8 June 2000 (2000-06-08)<br>* sequence 85 * | 1-15 | |

**TECHNICAL FIELDS
SEARCHED (IPC)**

The present search report has been drawn up for all claims

| Place of search | Date of completion of the search | Examiner |
|---|---|---|
| The Hague | 6 November 2023 | Schmitz, Till |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

  ...................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

**EP 23 30 6041**

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2023

| Patent document cited in search report | Publication date | Patent family member(s) | | Publication date |
|---|---|---|---|---|
| WO 2021189053 A1 | 23-09-2021 | EP | 4121583 A1 | 25-01-2023 |
| | | WO | 2021189053 A1 | 23-09-2021 |
| WO 0032221 A2 | 08-06-2000 | AT | E458050 T1 | 15-03-2010 |
| | | AU | 771751 B2 | 01-04-2004 |
| | | CA | 2347835 A1 | 08-06-2000 |
| | | EP | 1135485 A2 | 26-09-2001 |
| | | JP | 3695642 B2 | 14-09-2005 |
| | | JP | 3803681 B2 | 02-08-2006 |
| | | JP | 4358159 B2 | 04-11-2009 |
| | | JP | 2003524385 A | 19-08-2003 |
| | | JP | 2005046146 A | 24-02-2005 |
| | | JP | 2006006329 A | 12-01-2006 |
| | | KR | 20010086070 A | 07-09-2001 |
| | | MX | PA01005169 A | 02-07-2002 |
| | | WO | 0032221 A2 | 08-06-2000 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7214694 B
- WO 2017006296 A
- WO 2008011193 A2
- WO 2013163479 A1
- WO 2022024034 A1